# EUROPEAN PATENT SPECIFICATION

(11) **EP 2 157 953 B1**
(45) Date of publication and mention of the grant of the patent: **15.07.2015**
(21) Application number: 08763348.3
(22) Date of filing: 16.06.2008
(51) Int. Cl.: A61F 13/15

(54) **BETTER FITTING DISPOSABLE ABSORBENT ARTICLE WITH SUBSTANTIALLY CONTINUOUSLY DISTRIBUTED ABSORBENT PARTICULATE POLYMER MATERIAL**
EINWEGSAUGARTIKEL MIT EINEM SUBSTANTIELL KONTINUIERLICH VERTEILTEN SAUGFÄHIGEN POLYMERPARTIKELMATERIAL UND MIT VERBESSERTEM SITZ
ARTICLE ABSORBANT JETABLE À MEILLEURE ADAPTATION, AYANT UNE MATIÈRE POLYMÈRE PARTICULIÈRE ABSORBANTE DISTRIBUÉE DE FAÇON SENSIBLEMENT CONTINUE

(30) Priority: 18.06.2007 US 936085 P
(43) Date of publication of application: 03.03.2010
(73) Proprietor: The Procter & Gamble Company, Cincinnati, OH 45202 (US)
(72) Inventor: ASHTON, Gregory, Cincinnati, Ohio 45242 (US); HUNDORF, Harald, Hermann, D-53115 Bonn (DE); BERUDA, Holger, 65824 Schwalbach am Taunus (DE); BLESSING, Horst, Cincinnati, Ohio 45208 (US); DZIEZOK, Peter, 65239 Hochheim (DE); KRAUSE, Axel, 50374 Erftstadt (DE); SCHMIDT, Mattias, D-65510 Idstein (DE); STELZIG, Lutz, D-60489 Frankfurt Am Main (DE)
(74) Representative: Kremer, Véronique Marie Joséphine
(86) International application number: PCT/IB2008/052365
(87) International publication number: WO 2008/155710

(56) References cited:
- EP-A- 1 447 066
- WO-A-98/55062

## Description

### FIELD OF THE INVENTION

The present invention generally relates to an absorbent article, and more particularly to a disposable absorbent garments, such as a taped diaper or training pant, with absorbent particulate polymer material.

### BACKGROUND OF THE INVENTION

Absorbent articles, such as disposable diapers, training pants, and adult incontinence undergarments, absorb and contain body exudates. They also are intended to prevent body exudates from soiling, wetting, or otherwise contaminating clothing or other articles, such as bedding, that come in contact with the wearer. A disposable absorbent article, such as a disposable diaper, may be worn for several hours in a dry state or in a urine loaded state. Accordingly, efforts have been made toward improving the fit and comfort of the absorbent article to the wearer, both when the article is dry and when the article is fully or partially loaded with liquid exudate, while maintaining or enhancing the absorbing and containing functions of the article.

Some absorbent articles, like diapers, contain an absorbent polymer material (also known as super absorbent polymer), such as an absorbent particulate polymer material. Absorbent particulate polymer material absorbs liquid and swells and may be more effective when disposed in an absorbent article in a certain pattern or arrangement intended for optimal absorbency, fit, and/or comfort. Thus, it may be desirable for absorbent particulate polymer material to remain in its intended location in an absorbent article and absorbent particulate polymer material, therefore, is desirably immobilized in the absorbent article such that the absorbent particulate polymer material remains immobilized when the absorbent article is dry and when it is wet.

In addition to being absorbent, absorbent articles, such as diapers, desirably may be thin and flexible, for ease and comfort in use and for more convenient and neat packaging and storage. Absorbent articles, which may often be used in large quantities, may also desirably be inexpensive. Some technologies of immobilizing absorbent particulate polymer material in an absorbent article add bulk to the absorbent article and thereby increase thickness, reduce flexibility, and/or increase cost of the absorbent article. Other technologies for immobilizing absorbent particulate polymer material in an absorbent article may not be as effective in maintaining immobilization when the absorbent article is in a wet state as when in a dry state. Accordingly, there remains a need for a thin, flexible, and/or inexpensive absorbent article containing absorbent particulate polymer material with enhanced immobilization of the absorbent particulate polymer material in the article in dry and wet states. It would be furthermore desirable to meet this need without reducing, or even while enhancing, effective retention of the free liquid exudate before and during absorption by the absorbent core. By way of prior art, attention is particularly directed to EP-1447066 and WO98/55062, the preamble of claim 1 is based an the latest.

### SUMMARY OF THE INVENTION

The present invention addresses one or more technical problems described above and provides a disposable absorbent article is claimed herein which comprises a chassis and an absorbent core which comprises an absorbent particulate polymer material and which is substantially cellulose free. The chassis has end edges and longitudinal edges and contains a topsheet and a backsheet. The absorbent core is located between the topsheet and the backsheet. The chassis includes a pair of side panels joined with the chassis, the side panels extending laterally outwardly beyond the longitudinal edges, each side panel having at least one zone elastically extensible in a direction having a vector component in the lateral direction.

Other features and advantages of the invention may be apparent from reading the following detailed description, drawings, and claims.

### BRIEF DESCRIPTION OF THE DRAWINGS

Fig. 1 is a plan view of a diaper in accordance with an embodiment of the present invention.
Fig. 2 is a cross sectional view of the diaper shown in Fig. 1 taken along the sectional line 2-2 of Fig. 1.
Fig. 3 is a partial cross sectional view of an absorbent core layer in accordance with an embodiment of this invention.
Fig. 4 is a partial cross sectional view of an absorbent core layer in accordance with another embodiment of this invention.
Fig. 5 is a plan view of the absorbent core layer illustrated in Fig. 3.
Fig. 6 is a plan view of a second absorbent core layer in accordance with an embodiment of this invention.
Fig. 7a is a partial sectional view of an absorbent core comprising a combination of the first and second absorbent core layers illustrated in Figs. 5 and 6.
Fig. 7b is a partial sectional view of an absorbent core comprising a combination of the first and second absorbent core layers illustrated in Figs. 5 and 6
Fig. 8 is a plan view of the absorbent core illustrated in Figs. 7a and 7b.
Fig. 9 is a schematic representation of a rheometer.
Fig. 10 is a schematic illustration of a process for making an absorbent core in accordance with an embodiment of this invention.
Fig. 11 is a partial sectional view of an apparatus for making an absorbent core in accordance with an embodiment of this invention.
Fig. 12 is a perspective view of the printing roll illustrated in Fig. 11.
Fig. 13 is a partial sectional view of the printing roll illustrated in Fig. 12 showing an absorbent particulate polymer material reservoir.
Fig. 14 is a perspective view of the supporting roll illustrated in Fig. 12.
Fig. 15 is a plan view of a diaper in accordance with another embodiment of the present invention, with a cut-away portion to reveal underlying structure, the outer surface of the diaper facing the viewer.
Fig. 16 is a fragmentary plan view of an alternative embodiment of a side panel in accordance with one embodiment of the present invention.
Figs. 17A and 17B are plan views of the regions 17A and 17B, respectively, shown in Fig. 16, magnified to show the structural detail.

### DETAILED DESCRIPTION OF THE INVENTION

As summarized above, the present invention may encompass an absorbent article, such as a diaper, having absorbent core which may be substantially cellulose free. The combination of a core substantially cellulose free and side panels having elastically extensible zones advantageously may provide the disposable absorbent article with improved softness, flexibility, and conformity to a wearer's body for greater comfort without increasing the likelihood of leakage from the disposable absorbent article.

"Absorbent article" refers to devices that absorb and contain body exudates, and, more specifically, refers to devices that are placed against or in proximity to the body of the wearer to absorb and contain the various exudates discharged from the body. Absorbent articles may include diapers, training pants, adult incontinence undergarments, feminine hygiene products, breast pads, care mats, bibs, wound dressing products, and the like. As used herein, the term "body fluids" or "body exudates" includes, but is not limited to, urine, blood, vaginal discharges, breast milk, sweat and fecal matter.

"Absorbent core" means a structure typically disposed between a topsheet and backsheet of an absorbent article for absorbing and containing liquid received by the absorbent article and may comprise one or more substrates, absorbent polymer material disposed on the one or more substrates, and a thermoplastic composition on the absorbent particulate polymer material and at least a portion of the one or more substrates for immobilizing the absorbent particulate polymer material on the one or more substrates. In a multilayer absorbent core, the absorbent core may also include a cover layer. The one or more substrates and the cover layer may comprise a nonwoven. Further, the absorbent core is substantially cellulose free. The absorbent core does not include an acquisition system, a topsheet, or a backsheet of the absorbent article. In a certain embodiment, the absorbent core would consist essentially of the one or more substrates, the absorbent polymer material, the thermoplastic composition, and optionally the cover layer.

"Absorbent polymer material," "absorbent gelling material," "AGM," "superabsorbent," and "superabsorbent material" are used herein interchangeably and refer to cross linked polymeric materials that can absorb at least 5 times their weight of an aqueous 0.9% saline solution as measured using the Centrifuge Retention Capacity test (Edana 441.2-01).

"Absorbent particulate polymer material" is used herein to refer to an absorbent polymer material which is in particulate form so as to be flowable in the dry state.

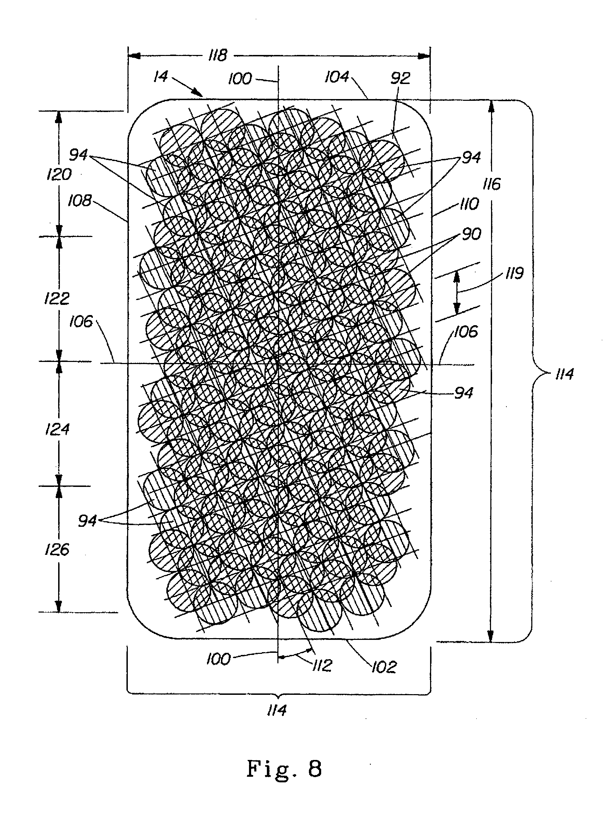

"Absorbent particulate polymer material area" as used herein refers to the area of the core wherein the first substrate 64 and second substrate 72 are separated by a multiplicity of superabsorbent particles. In Figure 8, the boundary of the absorbent particulate polymer material area is defined by the perimeter of the overlapping circles. There may be some extraneous superabsorbent particles outside of this perimeter between the first substrate 64 and second substrate 72.

"Airfelt" is used herein to refer to comminuted wood pulp, which is a form of cellulosic fiber.

"Comprise," "comprising," and "comprises" are open ended terms, each specifies the presence of what follows, e.g., a component, but does not preclude the presence of other features, e.g., elements, steps, components known in the art, or disclosed herein.

"Consisting essentially of" is used herein to limit the scope of subject matter, such as that in a claim, to the specified materials or steps and those that do not materially affect the basic and novel characteristics of the subject matter.

"Disposable" is used in its ordinary sense to mean an article that is disposed or discarded after a limited number of usage events over varying lengths of time, for example, less than about 20 events, less than about 10 events, less than about 5 events, or less than about 2 events.

"Diaper" refers to an absorbent article generally worn by infants and incontinent persons about the lower torso so as to encircle the waist and legs of the wearer and that is specifically adapted to receive and contain urinary and fecal waste. As used herein, term "diaper" also includes "pants" which is defined below.

"Elastic", "elastically extensible", and "elasticized" refer herein to the property of a material and/or an element of a diaper or other disposable absorbent article whereby the material and/or the element can be elongated to at least 150% of its original unstretched length without rupture or catastrophic failure upon the application of tensioning force and will substantially return to its original length or near its original length after the tension is released.

"Fiber" and "filament" are used interchangeably.

A "nonwoven" is a manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). Nonwoven fabrics can be formed by many processes such as meltblowing, spunbonding, solvent spinning, electrospinning, and carding. The basis weight of nonwoven fabrics is usually expressed in grams per square meter (gsm).

"Pant" or "training pant", as used herein, refer to disposable garments having a waist opening and leg openings designed for infant or adult wearers. A pant may be placed in position on the wearer by inserting the wearer's legs into the leg openings and sliding the pant into position about a wearer's lower torso. A pant may be preformed by any suitable technique including, but not limited to, joining together portions of the article using refastenable and/or non-refastenable bonds (e.g., seam, weld, adhesive, cohesive bond, fastener, etc.). A pant may be preformed anywhere along the circumference of the article (e.g., side fastened, front waist fastened). While the terms "pant" or "pants" are used herein, pants are also commonly referred to as "closed diapers," "prefastened diapers," "pull-on diapers," "training pants," and "diaper-pants". Suitable pants are disclosed in U.S. Patent No. 5,246,433, issued to Hasse, et al. on September 21, 1993; U.S. Patent No. 5,569,234, issued to Buell et al. on October 29, 1996; U.S. Patent No. 6,120,487, issued to Ashton on September 19, 2000; U.S. Patent No. 6,120,489, issued to Johnson et al. on September 19, 2000; U.S. Patent No. 4,940,464, issued to Van Gompel et al. on July 10, 1990; U.S. Patent No. 5,092,861, issued to Nomura et al. on March 3, 1992; U.S. Patent Publication No. 2003/0233082 A1, entitled "Highly Flexible And Low Deformation Fastening Device", filed on June 13, 2002; U.S. Patent No. 5,897,545, issued to Kline et al. on April 27, 1999; U.S. Patent No. 5,957,908, issued to Kline et al on September 28, 1999.

"Substantially cellulose free" is used herein to describe an article, such as an absorbent core, that contains less than 10% by weight cellulosic fibers, less than 5% cellulosic fibers, less than 1% cellulosic fibers, no cellulosic fibers, or no more than an immaterial amount of cellulosic fibers. An immaterial amount of cellulosic material would not materially affect the thinness, flexibility, or absorbency of an absorbent core.

"Substantially continuously distributed" as used herein indicates that within the absorbent particulate polymer material area, the first substrate 64 and second substrate 72 are separated by a multiplicity of superabsorbent particles. It is recognized that there may be minor incidental contact areas between the first substrate 64 and second substrate 72 within the absorbent particulate polymer material area. Incidental contact areas between the first substrate 64 and second substrate 72 may be intentional or unintentional (e.g. manufacturing artifacts) but do not form geometries such as pillows, pockets, tubes, quilted patterns and the like.

"Thermoplastic adhesive material" as used herein is understood to comprise a polymer composition from which fibers are formed and applied to the superabsorbent material with the intent to immobilize the superabsorbent material in both the dry and wet state. The thermoplastic adhesive material of the present invention forms a fibrous network over the superabsorbent material.

"Thickness" and "caliper" are used herein interchangeably.

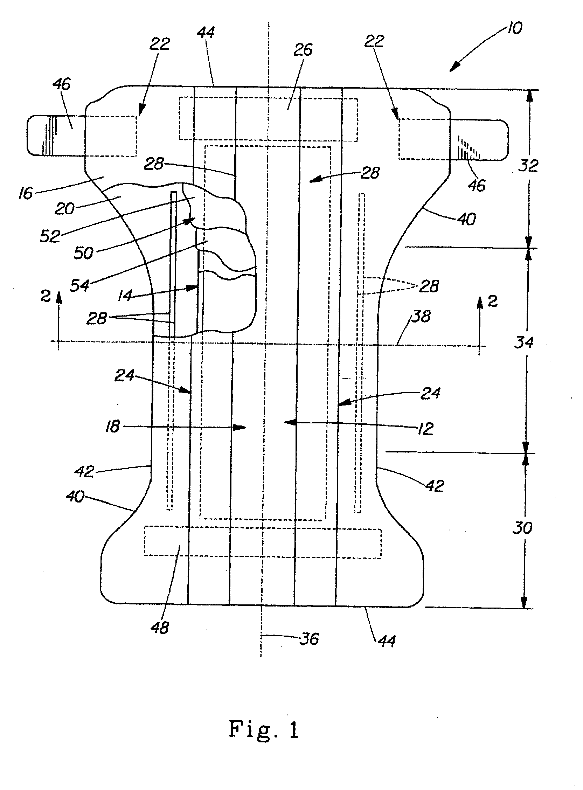

Fig. 1 is a plan view of a diaper 10 according to a certain embodiment of the present invention. The diaper 10 is shown in its flat out, uncontracted state (i.e., without elastic induced contraction) and portions of the diaper 10 are cut away to more clearly show the underlying structure of the diaper 10. A portion of the diaper 10 that contacts a wearer is facing the viewer in Fig. 1. The diaper 10 generally may comprise a chassis 12 and an absorbent core 14 disposed in the chassis.

The chassis 12 of the diaper 10 in Fig. 1 may comprise the main body of the diaper 10. The chassis 12 may comprise an outer covering 16 including a topsheet 18, which may be liquid pervious, and/or a backsheet 20, which may be liquid impervious. The absorbent core 14 may be encased between the topsheet 18 and the backsheet 20. The chassis 12 may also include side panels 22, elasticized leg cuffs 24, and an elastic waist feature 26.

The leg cuffs 24 and the elastic waist feature 26 may each typically comprise elastic members 28. One end portion of the diaper 10 may be configured as a first waist region 30 of the diaper 10. An opposite end portion of the diaper 10 may be configured as a second waist region 32 of the diaper 10. An intermediate portion of the diaper 10 may be configured as a crotch region 34, which extends longitudinally between the first and second waist regions 30 and 32. The waist regions 30 and 32 may include elastic elements such that they gather about the waist of the wearer to provide improved fit and containment (elastic waist feature 26). The crotch region 34 is that portion of the diaper 10 which, when the diaper 10 is worn, is generally positioned between the wearer's legs.

The diaper 10 is depicted in Fig. 1 with its longitudinal axis 36 and its transverse axis 38. The periphery 40 of the diaper 10 is defined by the outer edges of the diaper 10 in which the longitudinal edges 42 run generally parallel to the longitudinal axis 36 of the diaper 10 and the end edges 44 run between the longitudinal edges 42 generally parallel to the transverse axis 38 of the diaper 10. The chassis 12 may also comprise a fastening system, which may include at least one fastening member 46 and at least one stored landing zone 48.

The diaper 20 may also include such other features as are known in the art including front and rear ear panels, waist cap features, elastics and the like to provide better fit, containment and aesthetic characteristics. Such additional features are well known in the art and are e.g., described in U.S. Pat. No. 3,860,003 and U.S. Pat. No. 5,151,092.

In order to keep the diaper 10 in place about the wearer, at least a portion of the first waist region 30 may be attached by the fastening member 46 to at least a portion of the second waist region 32 to form leg opening(s) and an article waist. When fastened, the fastening system carries a tensile load around the article waist. The fastening system may allow an article user to hold one element of the fastening system, such as the fastening member 46, and connect the first waist region 30 to the second waist region 32 in at least two places. This may be achieved through manipulation of bond strengths between the fastening device elements.

According to certain embodiments, the diaper 10 may be provided with a re-closable fastening system or may alternatively be provided in the form of a pant-type diaper. When the absorbent article is a diaper, it may comprise a re-closable fastening system joined to the chassis for securing the diaper to a wearer. When the absorbent article is a pant-type diaper, the article may comprise at least two side panels joined to the chassis and to each other to form a pant. The fastening system and any component thereof may include any material suitable for such a use, including but not limited to plastics, films, foams, nonwoven, woven, paper, laminates, fiber reinforced plastics and the like, or combinations thereof. In certain embodiments, the materials making up the fastening device may be flexible. The flexibility may allow the fastening system to conform to the shape of the body and thus, reduce the likelihood that the fastening system will irritate or injure the wearer's skin.

For unitary absorbent articles, the chassis 12 and absorbent core 14 may form the main structure of the diaper 10 with other features added to form the composite diaper structure. While the topsheet 18, the backsheet 20, and the absorbent core 14 may be assembled in a variety of well-known configurations, preferred diaper configurations are described generally in U.S. Pat. No. 5,554,145 entitled "Absorbent Article With Multiple Zone Structural Elastic-Like Film Web Extensible Waist Feature" issued to Roe et al. on Sep. 10, 1996; U.S. Pat. No. 5,569,234 entitled "Disposable Pull-On Pant" issued to Buell et al. on Oct. 29, 1996; and U.S. Pat. No. 6,004,306 entitled "Absorbent Article With Multi-Directional Extensible Side Panels" issued to Robles et al. on Dec. 21, 1999.

The topsheet 18 in Fig. 1 may be fully or partially elasticized or may be foreshortened to provide a void space between the topsheet 18 and the absorbent core 14. Exemplary structures including elasticized or foreshortened topsheets are described in more detail in U.S. Pat. No. 5,037,416 entitled "Disposable Absorbent Article Having Elastically Extensible Topsheet" issued to Allen et al. on Aug. 6, 1991; and U.S. Pat. No. 5,269,775 entitled "Trisection Topsheets for Disposable Absorbent Articles and Disposable Absorbent Articles Having Such Trisection Topsheets" issued to Freeland et al. on Dec. 14, 1993.

The backsheet 26 may be joined with the topsheet 18. The backsheet 20 may prevent the exudates absorbed by the absorbent core 14 and contained within the diaper 10 from soiling other external articles that may contact the diaper 10, such as bed sheets and undergarments. In certain embodiments, the backsheet 26 may be substantially impervious to liquids (e.g., urine) and comprise a laminate of a nonwoven and a thin plastic film such as a thermoplastic film having a thickness of about 0.012 mm (0.5 mil) to about 0.051 mm (2.0 mils). Suitable backsheet films include those manufactured by Tredegar Industries Inc. of Terre Haute, Ind. and sold under the trade names X15306, X10962, and X10964. Other suitable backsheet materials may include breathable materials that permit vapors to escape from the diaper 10 while still preventing liquid exudates from passing through the backsheet 10. Exemplary breathable materials may include materials such as woven webs, nonwoven webs, composite materials such as film-coated nonwoven webs, and microporous films such as manufactured by Mitsui Toatsu Co., of Japan under the designation ESPOIR NO and by EXXON Chemical Co., of Bay City, Tex., under the designation EXXAIRE. Suitable breathable composite materials comprising polymer blends are available from Clopay Corporation, Cincinnati, Ohio under the name HYTREL blend P18-3097. Such breathable composite materials are described in greater detail in PCT Application No. WO 95/16746, published on Jun. 22, 1995 in the name of E. I. DuPont. Other breathable backsheets including nonwoven webs and apertured formed films are described in U.S. Pat. No. 5,571,096 issued to Dobrin et al. on Nov. 5, 1996.

In certain embodiments, the backsheet of the present invention may have a water vapor transmission rate (WVTR) of greater than about 2000 g/24h/m², greater than about 3000 g/24h/m², greater than about 5000 g/24h/m², greater than about 6000 g/24h/m², greater than about 7000 g/24h/m², greater than about 8000 g/24h/m², greater than about 9000 g/24h/m², greater than about 10000 g/24h/m², greater than about 11000 g/24h/m², greater than about 12000 g/24h/m², greater than about 15000 g/24h/m², measured according to WSP 70.5 (08) at 37.8 °C and 60% Relative Humidity.

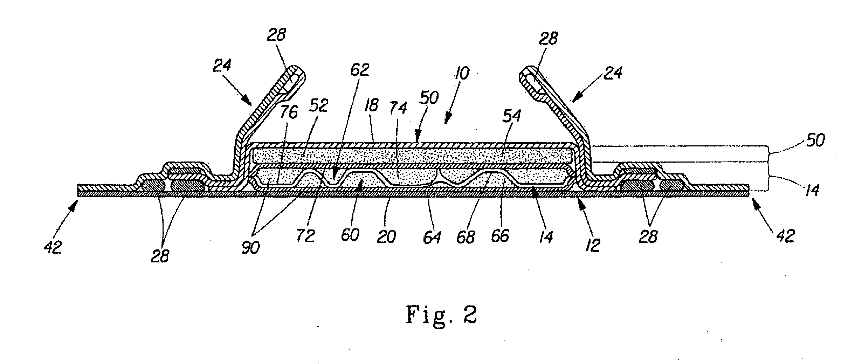

Fig. 2 shows a cross section of Fig. 1 taken along the sectional line 2-2 of Fig. 1. Starting from the wearer facing side, the diaper 10 may comprise the topsheet 18, the components of the absorbent core 14, and the backsheet 20. According to a certain embodiment, diaper 10 may also comprise an acquisition system 50 disposed between the liquid permeable topsheet 18 and a wearer facing side of the absorbent core 14. The acquisition system 50 may be in direct contact with the absorbent core. The acquisition system 50 may comprise a single layer or multiple layers, such as an upper acquisition layer 52 facing towards the wearer's skin and a lower acquisition 54 layer facing the garment of the wearer. According to a certain embodiment, the acquisition system 50 may function to receive a surge of liquid, such as a gush of urine. In other words, the acquisition system 50 may serve as a temporary reservoir for liquid until the absorbent core 14 can absorb the liquid.

In a certain embodiment, the acquisition system 50 may comprise chemically cross-linked cellulosic fibers. Such cross-linked cellulosic fibers may have desirable absorbency properties. Exemplary chemically cross-linked cellulosic fibers are disclosed in US Patent No. 5,137,537. In certain embodiments, the chemically cross-linked cellulosic fibers are cross-linked with between about 0.5 mole % and about 10.0 mole % of a C₂ to C₉ polycarboxylic cross-linking agent or between about 1.5 mole % and about 6.0 mole % of a C₂ to C₉ polycarboxylic cross-linking agent based on glucose unit. Citric acid is an exemplary cross-linking agent. In other embodiments, polyacrylic acids may be used. Further, according to certain embodiments, the cross-linked cellulosic fibers have a water retention value of about 25 to about 60, or about 28 to about 50, or about 30 to about 45. A method for determining water retention value is disclosed in US Patent No. 5,137,537. According to certain embodiments, the cross-linked cellulosic fibers may be crimped, twisted, or curled, or a combination thereof including crimped, twisted, and curled.

In a certain embodiment, one or both of the upper and lower acquisition layers 52 and 54 may comprise a non-woven, which may be hydrophilic. Further, according to a certain embodiment, one or both of the upper and lower acquisition layers 52 and 54 may comprise the chemically cross-linked cellulosic fibers, which may or may not form part of a nonwoven material. According to an exemplary embodiment, the upper acquisition layer 52 may comprise a nonwoven, without the cross-linked cellulosic fibers, and the lower acquisition layer 54 may comprise the chemically cross-linked cellulosic fibers. Further, according to an embodiment, the lower acquisition layer 54 may comprise the chemically cross-linked cellulosic fibers mixed with other fibers such as natural or synthetic polymeric fibers. According to exemplary embodiments, such other natural or synthetic polymeric fibers may include high surface area fibers, thermoplastic binding fibers, polyethylene fibers, polypropylene fibers, PET fibers, rayon fibers, lyocell fibers, and mixtures thereof. According to a particular embodiment, the lower acquisition layer 54 has a total dry weight, the cross-linked cellulosic fibers are present on a dry weight basis in the upper acquisition layer in an amount from about 30 % to about 95 % by weight of the lower acquisition layer 54, and the other natural or synthetic polymeric fibers are present on a dry weight basis in the lower acquisition layer 54 in an amount from about 70 % to about 5 % by weight of the lower acquisition layer 54. According to another embodiment, the cross-linked cellulosic fibers are present on a dry weight basis in the first acquisition layer in an amount from about 80 % to about 90 % by weight of the lower acquisition layer 54, and the other natural or synthetic polymeric fibers are present on a dry weight basis in the lower acquisition layer 54 in an amount from about 20 % to about 10 % by weight of the lower acquisition layer 54.

According to a certain embodiment, the lower acquisition layer 54 desirably has a high fluid uptake capability. Fluid uptake is measured in grams of absorbed fluid per gram of absorbent material and is expressed by the value of "maximum uptake." A high fluid uptake corresponds therefore to a high capacity of the material and is beneficial, because it ensures the complete acquisition of fluids to be absorbed by an acquisition material. According to exemplary embodiments, the lower acquisition layer 54 has a maximum uptake of about 10 g/g.

A relevant attribute of the upper acquisition layer 54 is its Median Desorption Pressure, MDP. The MDP is a measure of the capillary pressure that is required to dewater the lower acquisition layer 54 to about 50% of its capacity at 0 cm capillary suction height under an applied mechanical pressure of 0.3psi. Generally, a relatively lower MDP may be useful. The lower MDP may allow the lower acquisition layer 54 to more efficiently drain the upper acquisition material. Without wishing to be bound by theory, a given distribution material may have a definable capillary suction. The ability of the lower acquisition layer 54 to move liquid vertically via capillary forces will be directly impacted by gravity and the opposing capillary forces associated with desorption of the upper acquisition layer. Minimizing these capillary forces may positively impact the performance of the lower acquisition layer 54. However, in a certain embodiment the lower acquisition layer 54 may also have adequate capillary absorption suction in order to drain the layers above (upper acquisition layer 52 and topsheet 18, in particular) and to temporarily hold liquid until the liquid can be partitioned away by the absorbent core components. Therefore, in a certain embodiment, the lower acquisition layer 54 may have a minimum MDP of greater than 5 cm. Further, according to exemplary embodiments, the lower acquisition layer 54 has an MDP value of less than about 20.5 cm H₂O, or less than about 19 cm H₂O, or less than about 18 cm H₂O to provide for fast acquisition.

The methods for determining MDP and maximum uptake are disclosed in U.S. Patent Application 11/600,691 (Flohr et al.). For example, according to a first embodiment, the lower acquisition layer 54 may comprise about 70 % by weight of chemically cross-linked cellulose fibers, about 10 % by weight polyester (PET), and about 20 % by weight untreated pulp fibers. According to a second embodiment, the lower acquisition layer 54 may comprise about 70 % by weight chemically cross-linked cellulose fibers, about 20 % by weight lyocell fibers, and about 10% by weight PET fibers. According to a third embodiment, the lower acquisition layer 54 may comprise about 68 % by weight chemically cross-linked cellulose fibers, about 16 % by weight untreated pulp fibers, and about 16 % by weight PET fibers. In one embodiment, the lower acquisition layer 54 may comprise from about 90-100% by weight chemically cross-linked cellulose fibers.

Suitable non-woven materials for the upper and lower acquisition layers 52 and 54 include, but are not limited to SMS material, comprising a spunbonded, a melt-blown and a further spunbonded layer. In certain embodiments, permanently hydrophilic non-wovens, and in particular, nonwovens with durably hydrophilic coatings are desirable. Another suitable embodiment comprises a SMMS-structure. In certain embodiments, the non-wovens are porous.

In certain embodiments, suitable non-woven materials may include, but are not limited to synthetic fibers, such as PE, PET, and PP. As polymers used for nonwoven production may be inherently hydrophobic, they may be coated with hydrophilic coatings. One way to produce nonwovens with durably hydrophilic coatings, is via applying a hydrophilic monomer and a radical polymerization initiator onto the nonwoven, and conducting a polymerization activated via UV light resulting in monomer chemically bound to the surface of the nonwoven as described in co-pending U.S. Patent Publication No. 2005/0159720. Another way to produce nonwovens with durably hydrophilic coatings is to coat the nonwoven with hydrophilic nanoparticles as described in co-pending applications U.S. Patent No. 7,112,621 to Rohrbaugh et al. and in PCT Application Publication WO 02/064877.

Typically, nanoparticles have a largest dimension of below 750 nm. Nanoparticles with sizes ranging from 2 to 750 nm may be economically produced. An advantage of nanoparticles is that many of them can be easily dispersed in water solution to enable coating application onto the nonwoven, they typically form transparent coatings, and the coatings applied from water solutions are typically sufficiently durable to exposure to water. Nanoparticles can be organic or inorganic, synthetic or natural. Inorganic nanoparticles generally exist as oxides, silicates, and/or, carbonates. Typical examples of suitable nanoparticles are layered clay minerals (e.g., LAPONITE™ from Southern Clay Products, Inc. (USA), and Boehmite alumina (e.g., Disperal P2™ from North American Sasol. Inc.). According to a certain embodiment, a suitable nanoparticle coated non-woven is that disclosed in the co-pending patent application Ser. No. 10/758,066 entitled "Disposable absorbent article comprising a durable hydrophilic core wrap" to Ekaterina Anatolyevna Ponomarenko and Mattias NMN Schmidt.

Further useful non-wovens are described in U.S. Pat. No. 6,645,569 to Cramer et al., U.S. Patent No. 6,863,933 to Cramer et al., U.S. Patent No. 7,112,621 to Rohrbaugh et al., and co-pending patent applications 10/338,603 to Cramer et al. and 10/338,610 to Cramer et al.

In some cases, the nonwoven surface can be pre-treated with high energy treatment (corona, plasma) prior to application of nanoparticle coatings. High energy pre-treatment typically temporarily increases the surface energy of a low surface energy surface (such as PP) and thus enables better wetting of a nonwoven by the nanoparticle dispersion in water.

Notably, permanently hydrophilic non-wovens are also useful in other parts of an absorbent article. For example, topsheets and absorbent core layers comprising permanently hydrophilic non-wovens as described above have been found to work well.

According to a certain embodiment, the upper acquisition layer 52 may comprise a material that provides good recovery when external pressure is applied and removed. Further, according to a certain embodiment, the upper acquisition layer 52 may comprise a blend of different fibers selected, for example from the types of polymeric fibers described above. In some embodiments, at least a portion of the fibers may exhibit a spiral-crimp which has a helical shape. In some embodiments, the upper acquisition layer 52 may comprise fibers having different degrees or types of crimping, or both. For example, one embodiment may include a mixture of fibers having about 8 to about 12 crimps per inch (cpi) or about 9 to about 10 cpi, and other fibers having about 4 to about 8 cpi or about 5 to about 7 cpi. Different types of crimps include, but are not limited to a 2D crimp or "flat crimp" and a 3D or spiral-crimp. According to a certain embodiment, the fibers may include bi-component fibers, which are individual fibers each comprising different materials, usually a first and a second polymeric material. It is believed that the use of side-by-side bi-component fibers is beneficial for imparting a spiral-crimp to the fibers.

The upper acquisition layer 52 may be stabilized by a latex binder, for example a styrenebutadiene latex binder (SB latex), in a certain embodiment. Processes for obtaining such lattices are known, for example, from EP 149 880 (Kwok) and US 2003/0105190 (Diehl et al.). In certain embodiments, the binder may be present in the upper acquisition layer 52 in excess of about 12%, about 14% or about 16% by weight. For certain embodiments, SB latex is available under the trade name GENFLO™ 3160 (OMNOVA Solutions Inc.; Akron, Ohio).

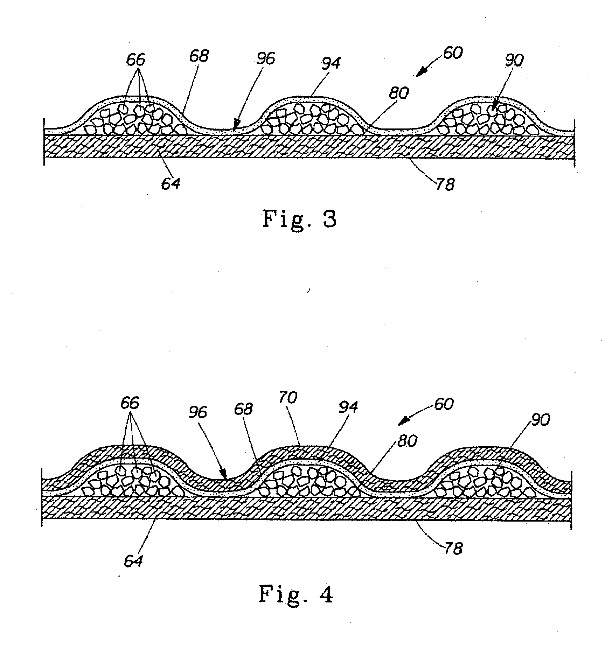

The absorbent core 14 in Figs. 1-8 generally is disposed between the topsheet 18 and the backsheet 20 and comprises two layers, a first absorbent layer 60 and a second absorbent layer 62. As best shown in Fig. 3, the first absorbent layer 60 of the absorbent core 14 comprises a substrate 64, an absorbent particular polymer material 66 on the substrate 64, and a thermoplastic composition 68 on the absorbent particulate polymer material 66 and at least portions of the first substrate 64 as an adhesive for covering and immobilizing the absorbent particulate polymer material 66 on the first substrate 64. According to another embodiment illustrated in Fig. 4, the first absorbent layer 60 of the absorbent core 14 may also include a cover layer 70 on the thermoplastic composition 68.

Likewise, as best illustrated in Fig. 2, the second absorbent layer 62 of the absorbent core 14 also includes a substrate 72, an absorbent particulate polymer material 74 on the second substrate 72, and a thermoplastic composition 66 on the absorbent particulate polymer material 74 and at least a portion of the second substrate 72 for immobilizing the absorbent particulate polymer material 74 on the second substrate 72. Although not illustrated, the second absorbent layer 62 may also include a cover layer such as the cover layer 70 illustrated in Fig. 4.

The substrate 64 of the first absorbent layer 60 may be referred to as a dusting layer and has a first surface 78 which faces the backsheet 20 of the diaper 10 and a second surface 80 which faces the absorbent particulate polymer material 66. Likewise, the substrate 72 of the second absorbent layer 62 may be referred to as a core cover and has a first surface 82 facing the topsheet 18 of the diaper 10 and a second surface 84 facing the absorbent particulate polymer material 74. The first and second substrates 64 and 72 may be adhered to one another with adhesive about the periphery to form an envelope about the absorbent particulate polymer materials 66 and 74 to hold the absorbent particulate polymer material 66 and 74 within the absorbent core 14.

According to a certain embodiment, the substrates 64 and 72 of the first and second absorbent layers 60 and 62 may be a non-woven material, such as those nonwoven materials described above. In certain embodiments, the non-wovens are porous and in one embodiment has a pore size of about 32 microns.

As illustrated in Figs. 1-8, the absorbent particulate polymer material 66 and 74 is deposited on the respective substrates 64 and 72 of the first and second absorbent layers 60 and 62 in clusters 90 of particles to form a grid pattern 92 comprising land areas 94 and junction areas 96 between the land areas 94. As defined herein, land areas 94 are areas where the thermoplastic adhesive material does not contact the nonwoven substrate or the auxiliary adhesive directly; junction areas 96 are areas where the thermoplastic adhesive material does contact the nonwoven substrate or the auxiliary adhesive directly. The junction areas 96 in the grid pattern 92 contain little or no absorbent particulate polymer material 66 and 74. The land areas 94 and junction areas 96 can have a variety of shapes including, but not limited to, circular, oval, square, rectangular, triangular, and the like.

The grid pattern shown in Fig. 8 is a square grid with regular spacing and size of the land areas. Other grid patterns including hexagonal, rhombic, orthorhombic, parallelogram, triangular, rectangular, and combinations thereof may also be used. The spacing between the grid lines may be regular or irregular.

The size of the land areas 94 in the grid patterns 92 may vary. According to certain embodiments, the width 119 of the land areas 94 in the grid patterns 92 ranges from about 8mm to about 12mm. In a certain embodiment, the width of the land areas 94 is about 10mm. The junction areas 96, on the other hand, in certain embodiments, have a width or larger span of less than about 5mm, less than about 3mm, less than about 2mm, less than about 1.5mm, less than about 1mm, or less than about 0.5mm.

As shown in Fig. 8, the absorbent core 14 has a longitudinal axis 100 extending from a rear end 102 to a front end 104 and a transverse axis 106 perpendicular to the longitudinal axis 100 extending from a first edge 108 to a second edge 110. The grid pattern 92 of absorbent particulate polymer material clusters 90 is arranged on the substrates 64 and 72 of the respective absorbent layers 60 and 62 such that the grid pattern 92 formed by the arrangement of land areas 94 and junction areas 96 forms a pattern angle 112. The pattern angle 112 may be 0, greater than 0, or 15 to 30 degrees, or from about 5 to about 85 degrees, or from about 10 to about 60 degrees, or from about 15 to about 30 degrees.

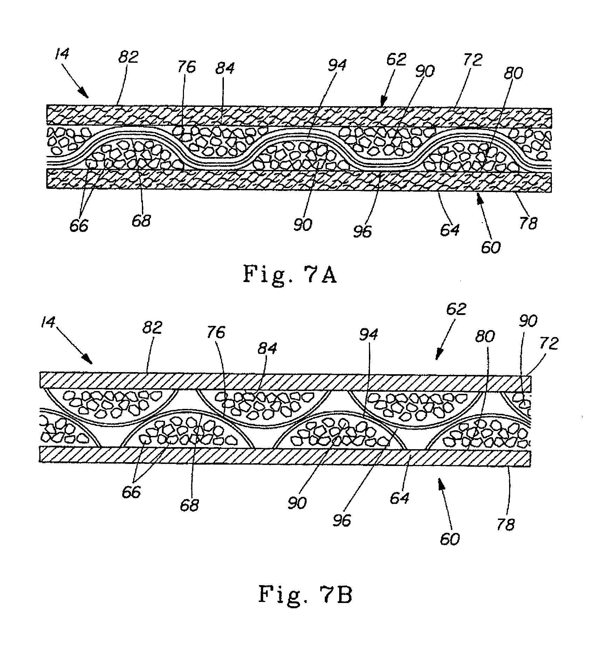

As best seen in Figs. 7a, 7b, and 8, the first and second layers 60 and 62 are combined to form the absorbent core 14. The absorbent core 14 has an absorbent particulate polymer material area 114 bounded by a pattern length 116 and a pattern width 118. The extent and shape of the absorbent particulate polymer material area 114 may vary depending on the desired application of the absorbent core 14 and the particular absorbent article in which it may be incorporated. In a certain embodiment, however, the absorbent particulate polymer material area 114 extends substantially entirely across the absorbent core 14, such as is illustrated in Fig. 8.

The first and second absorbent layers 60 and 62 are combined together to form the absorbent core 14 such that the grid patterns 92 of the respective first and second absorbent layers 62 and 64 are offset from one another along the length and/or width of the absorbent core 14. The respective grid patterns 92 are offset such that the absorbent particulate polymer material 66 and 74 is substantially continuously distributed across the absorbent particulate polymer area 114. The absorbent particulate polymer material 66 and 74 is substantially continuously distributed across the absorbent particulate polymer material area 114 despite the individual grid patterns 92 comprising absorbent particulate polymer material 66 and 74 discontinuously distributed across the first and second substrates 64 and 72 in clusters 90. In a certain embodiment, the grid patterns may be offset such that the land areas 94 of the first absorbent layer 60 face the junction areas 96 of the second absorbent layer 62 and the land areas of the second absorbent layer 62 face the junction areas 96 of the first absorbent layer 60. When the land areas 94 and junction areas 96 are appropriately sized and arranged, the resulting combination of absorbent particulate polymer material 66 and 74 is a substantially continuous layer of absorbent particular polymer material across the absorbent particulate polymer material area 114 of the absorbent core 14 (i.e. first and second substrates 64 and 72 do not form a plurality of pockets, each containing a cluster 90 of absorbent particulate polymer material 66 therebetween). In a certain embodiment, respective grid patterns 92 of the first and second absorbent layer 60 and 62 may be substantially the same.

In a certain embodiment as illustrated in Fig. 8, the amount of absorbent particulate polymer material 66 and 74 may vary along the length 116 of the grid pattern 92. In a certain embodiment, the grid pattern may be divided into absorbent zones 120, 122, 124, and 126, in which the amount of absorbent particulate polymer material 66 and 74 varies from zone to zone. As used herein, "absorbent zone" refers to a region of the absorbent particulate polymer material area having boundaries that are perpendicular to the longitudinal axis shown in Fig. 8. The amount of absorbent particulate polymer material 66 and 74 may, in a certain embodiment, gradually transition from one of the plurality of absorbent zones 120, 122, 124, and 126 to another. This gradual transition in amount of absorbent particulate polymer material 66 and 74 may reduce the possibility of cracks forming in the absorbent core 14.

The amount of absorbent particulate polymer material 66 and 74 present in the absorbent core 14 may vary, but in certain embodiments, is present in the absorbent core in an amount greater than about 80% by weight of the absorbent core, or greater than about 85% by weight of the absorbent core, or greater than about 90% by weight of the absorbent core, or greater than about 95% by weight of the core. In a particular embodiment, the absorbent core 14 consists essentially of the first and second substrates 64 and 72, the absorbent particulate polymer material 66 and 74, and the thermoplastic adhesive composition 68 and 76. The absorbent core 14 is substantially cellulose free.

According to certain embodiments, the weight of absorbent particulate polymer material 66 and 74 in at least one freely selected first square measuring 1 cm x 1 cm may be at least about 10%, or 20%, or 30%, 40% or 50% higher than the weight of absorbent particulate polymer material 66 and 74 in at least one freely selected second square measuring 1 cm x 1 cm. In a certain embodiment, the first and the second square are centered about the longitudinal axis.

The absorbent particulate polymer material area, according to an exemplary embodiment, may have a relatively narrow width in the crotch area of the absorbent article for increased wearing comfort. Hence, the absorbent particulate polymer material area, according to an embodiment, may have a width as measured along a transverse line which is positioned at equal distance to the front edge and the rear edge of the absorbent article, which is less than about 100 mm, 90 mm, 80 mm, 70 mm, 60 mm or even less than about 50 mm.

It has been found that, for most absorbent articles such as diapers, the liquid discharge occurs predominately in the front half of the diaper. The front half of the absorbent core 14 should therefore comprise most of the absorbent capacity of the core. Thus, according to certain embodiments, the front half of said absorbent core 14 may comprise more than about 60% of the superabsorbent material, or more than about 65%, 70%, 75%, 80%, 85%, or 90% of the superabsorbent material.

In certain embodiments, the absorbent core 14 may further comprise any absorbent material that is generally compressible, conformable, non-irritating to the wearer's skin, and capable of absorbing and retaining liquids such as urine and other certain body exudates. In such embodiments, the absorbent core 14 may comprise a wide variety of liquid-absorbent materials commonly used in disposable diapers and other absorbent articles such as comminuted wood pulp, which is generally referred to as airfelt, creped cellulose wadding, melt blown polymers, including co-form, chemically stiffened, modified or cross-linked cellulosic fibers, tissue, including tissue wraps and tissue laminates, absorbent foams, absorbent sponges, or any other known absorbent material or combinations of materials. The absorbent core 14 may further comprise minor amounts (typically less than about 10%) of materials, such as adhesives, waxes, oils and the like.

Exemplary absorbent structures for use as the absorbent assemblies are described in U.S. Pat. No. 4,610,678 (Weisman et al.); U.S. Pat. No. 4,834,735 (Alemany et al.); U.S. Pat. No. 4,888,231 (Angstadt); U.S. Pat. No. 5,260,345 (DesMarais et al.); U.S. Pat. No. 5,387,207 (Dyer et al.); U.S. Pat. No. 5,397,316 (LaVon et al.); and U.S. Pat. No. 5,625,222 (DesMarais et al.).

The thermoplastic adhesive material 68 and 76 may serve to cover and at least partially immobilize the absorbent particulate polymer material 66 and 74. In one embodiment of the present invention, the thermoplastic adhesive material 68 and 76 can be disposed essentially uniformly within the absorbent particulate polymer material 66 and 74, between the polymers. However, in a certain embodiment, the thermoplastic adhesive material 68 and 76 may be provided as a fibrous layer which is at least partially in contact with the absorbent particulate polymer material 66 and 74 and partially in contact with the substrate layers 64 and 72 of the first and second absorbent layers 60 and 62. Figs. 3, 4, and 7 show such a structure, and in that structure, the absorbent particulate polymer material 66 and 74 is provided as a discontinuous layer, and a layer of fibrous thermoplastic adhesive material 68 and 76 is laid down onto the layer of absorbent particulate polymer material 66 and 74, such that the thermoplastic adhesive material 68 and 76 is in direct contact with the absorbent particulate polymer material 66 and 74, but also in direct contact with the second surfaces 80 and 84 of the substrates 64 and 72, where the substrates are not covered by the absorbent particulate polymer material 66 and 74. This imparts an essentially three-dimensional structure to the fibrous layer of thermoplastic adhesive material 68 and 76, which in itself is essentially a two-dimensional structure of relatively small thickness, as compared to the dimension in length and width directions. In other words, the thermoplastic adhesive material 68 and 76 undulates between the absorbent particulate polymer material 68 and 76 and the second surfaces of the substrates 64 and 72.

Thereby, the thermoplastic adhesive material 68 and 76 may provide cavities to cover the absorbent particulate polymer material 66 and 74, and thereby immobilizes this material. In a further aspect, the thermoplastic adhesive material 68 and 76 bonds to the substrates 64 and 72 and thus affixes the absorbent particulate polymer material 66 and 74 to the substrates 64 and 72. Thus, in accordance with certain embodiments, the thermoplastic adhesive material 68 and 76 immobilizes the absorbent particulate polymer material 66 and 74 when wet, such that the absorbent core 14 achieves an absorbent particulate polymer material loss of no more than about 70%, 60%, 50%, 40%, 30%, 20%, 10% according to the Wet Immobilization Test described herein. Some thermoplastic adhesive materials will also penetrate into both the absorbent particulate polymer material 66 and 74 and the substrates 64 and 72, thus providing for further immobilization and affixation. Of course, while the thermoplastic adhesive materials disclosed herein provide a much improved wet immobilization (i.e., immobilization of absorbent material when the article is wet or at least partially loaded), these thermoplastic adhesive materials may also provide a very good immobilization of absorbent material when the absorbent core 14 is dry. The thermoplastic adhesive material 68 and 76 may also be referred to as a hot melt adhesive.

Without wishing to be bound by theory, it has been found that those thermoplastic adhesive materials which are most useful for immobilizing the absorbent particulate polymer material 66 and 74 combine good cohesion and good adhesion behavior. Good adhesion may promote good contact between the thermoplastic adhesive material 68 and 76 and the absorbent particulate polymer material 66 and 74 and the substrates 64 and 72. Good cohesion reduces the likelihood that the adhesive breaks, in particular in response to external forces, and namely in response to strain. When the absorbent core 14 absorbs liquid, the absorbent particulate polymer material 66 and 74 swells and subjects the thermoplastic adhesive material 68 and 76 to external forces. In certain embodiments, the thermoplastic adhesive material 68 and 76 may allow for such swelling, without breaking and without imparting too many compressive forces, which would restrain the absorbent particulate polymer material 66 and 74 from swelling.

In accordance with certain embodiments, the thermoplastic adhesive material 68 and 76 may comprise, in its entirety, a single thermoplastic polymer or a blend of thermoplastic polymers, having a softening point, as determined by the ASTM Method D-36-95 "Ring and Ball", in the range between 50 °C and 300 °C, or alternatively the thermoplastic adhesive material may be a hot melt adhesive comprising at least one thermoplastic polymer in combination with other thermoplastic diluents such as tackifying resins, plasticizers and additives such as antioxidants. In certain embodiments, the thermoplastic polymer has typically a molecular weight (Mw) of more than 10,000 and a glass transition temperature (Tg) usually below room temperature or -6 °C > Tg < 16°C. In certain embodiments, typical concentrations of the polymer in a hot melt are in the range of about 20 to about 40% by weight. In certain embodiments, thermoplastic polymers may be water insensitive. Exemplary polymers are (styrenic) block copolymers including A-B-A triblock structures, A-B diblock structures and (A-B)n radial block copolymer structures wherein the A blocks are non-elastomeric polymer blocks, typically comprising polystyrene, and the B blocks are unsaturated conjugated diene or (partly) hydrogenated versions of such. The B block is typically isoprene, butadiene, ethylene/butylene (hydrogenated butadiene), ethylene/propylene (hydrogenated isoprene), and mixtures thereof.

Other suitable thermoplastic polymers that may be employed are metallocene polyolefins, which are ethylene polymers prepared using single-site or metallocene catalysts. Therein, at least one comonomer can be polymerized with ethylene to make a copolymer, terpolymer or higher order polymer. Also applicable are amorphous polyolefins or amorphous polyalphaolefins (APAO) which are homopolymers, copolymers or terpolymers of C2 to C8 alpha olefins.

In exemplary embodiments, the tackifying resin has typically a Mw below 5,000 and a Tg usually above room temperature, typical concentrations of the resin in a hot melt are in the range of about 30 to about 60%, and the plasticizer has a low Mw of typically less than 1,000 and a Tg below room temperature, with a typical concentration of about 0 to about 15%.

In certain embodiments, the thermoplastic adhesive material 68 and 76 is present in the form of fibers. In some embodiments, the fibers will have an average thickness of about 1 to about 50 micrometers or about 1 to about 35 micrometers and an average length of about 5 mm to about 50 mm or about 5mm to about 30 mm. To improve the adhesion of the thermoplastic adhesive material 68 and 76 to the substrates 64 and 72 or to any other layer, in particular any other non-woven layer, such layers may be pre-treated with an auxiliary adhesive.

In certain embodiments, the thermoplastic adhesive material 68 and 76 will meet at least one, or several, or all of the following parameters:
An exemplary thermoplastic adhesive material 68 and 76 may have a storage modulus G' measured at 20°C of at least 30,000 Pa and less than 300,000 Pa, or less than 200,000 Pa, or between 140,000 Pa and 200,000 Pa, or less than 100,000 Pa. In a further aspect, the storage modulus G' measured at 35°C may be greater than 80,000 Pa. In a further aspect, the storage modulus G' measured at 60°C may be less than 300,000 Pa and more than 18,000 Pa, or more than 24,000 Pa, or more than 30,000Pa, or more than 90,000 Pa. In a further aspect, the storage modulus G' measured at 90°C may be less than 200,000 Pa and more than 10,000 Pa, or more than 20,000 Pa, or more then 30,000Pa. The storage modulus measured at 60°C and 90°C may be a measure for the form stability of the thermoplastic adhesive material at elevated ambient temperatures. This value is particularly important if the absorbent product is used in a hot climate where the thermoplastic adhesive material would lose its integrity if the storage modulus G' at 60°C and 90 °C is not sufficiently high.

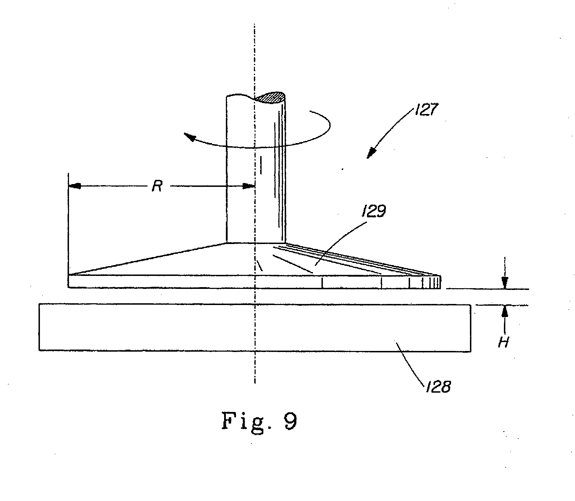

G' is measured using a rheometer as schematically shown in Fig. 9 for the purpose of general illustration only. The rheometer 127 is capable of applying a shear stress to the adhesive and measuring the resulting strain (shear deformation) response at constant temperature. The adhesive is placed between a Peltier-element acting as lower, fixed plate 128 and an upper plate 129 with a radius R of e.g., 10 mm, which is connected to the drive shaft of a motor to generate the shear stress. The gap between both plates has a height H of e.g., 1500 micron. The Peltier-element enables temperature control of the material (+0.5°C). The strain rate and frequency should be chosen such that all measurements are made in the linear viscoelastic region.

The absorbent core 14 may also comprise an auxiliary adhesive which is not illustrated in the figures. The auxiliary adhesive may be deposited on the first and second substrates 64 and 72 of the respective first and second absorbent layers 60 and 62 before application of the absorbent particulate polymer material 66 and 74 for enhancing adhesion of the absorbent particulate polymer materials 66 and 74 and the thermoplastic adhesive material 68 and 76 to the respective substrates 64 and 72. The auxiliary glue may also aid in immobilizing the absorbent particulate polymer material 66 and 74 and may comprise the same thermoplastic adhesive material as described hereinabove or may also comprise other adhesives including but not limited to sprayable hot melt adhesives, such as H.B. Fuller Co. (St. Paul, MN) Product No. HL-1620-B. The auxiliary glue may be applied to the substrates 64 and 72 by any suitable means, but according to certain embodiments, may be applied in about 0.5 to about 1mm wide slots spaced about 0.5 to about 2 mm apart.

The cover layer 70 shown in Fig. 4 may comprise the same material as the substrates 64 and 72, or may comprise a different material. In certain embodiments, suitable materials for the cover layer 70 are the non-woven materials, typically the materials described above as useful for the substrates 64 and 72.

In another aspect of the present invention, a disposable absorbent article is provided which comprises a chassis, having end edges and longitudinal edges, and including (i) a topsheet, (ii) a backsheet, and (iii) an absorbent core located between the topsheet and the backsheet, wherein the absorbent core comprises an absorbent particulate polymer material and is substantially cellulose free. A pair of side panels is joined with the chassis, the side panels extending laterally outwardly beyond the longitudinal edges, each side panel having at least one zone that is elastically extensible in a direction having a vector component in the lateral direction, to provide improved fit and containment. In a certain embodiment, the extensible side panels provide a better fitting taped diaper or pant, which advantageously may provide improved gasketing that will allow time for the substantially cellulose free absorbent core to absorb urine and other liquid exudates.

A portion or the whole of the chassis may be made extensible to a degree greater than the inherent extensibility of the material or materials from which the chassis is made. The additional extensibility may be desirable in order to allow the chassis to conform to the body of a wearer during movement by the wearer. The additional extensibility may also be desirable, for example, in order to allow the user of a diaper including a chassis having a particular size before extension to extend the front waist region, the back waist region, or both waist regions of the chassis to encircle the waist of an individual wearer whose waist circumference falls within a predefined range, i.e., to tailor the diaper to the individual wearer. Such extension of the waist region or regions may give the diaper a generally hourglass shape, so long as the crotch region is extended to a relatively lesser degree than the waist region or regions, and may impart a tailored appearance to the diaper when it is worn. In addition, a lesser amount of material may be needed in order to make a diaper capable of being properly fit onto a given size of a wearer when the material is made extensible as described.

The side panels, which may be referred to as elastically stretchable ear panels, may be separate parts made of elastic material, which are attached to the longitudinal edges of the chassis in the back and/or front waist region. Alternatively, the side panels may be an integral part of the backsheet or topsheet.

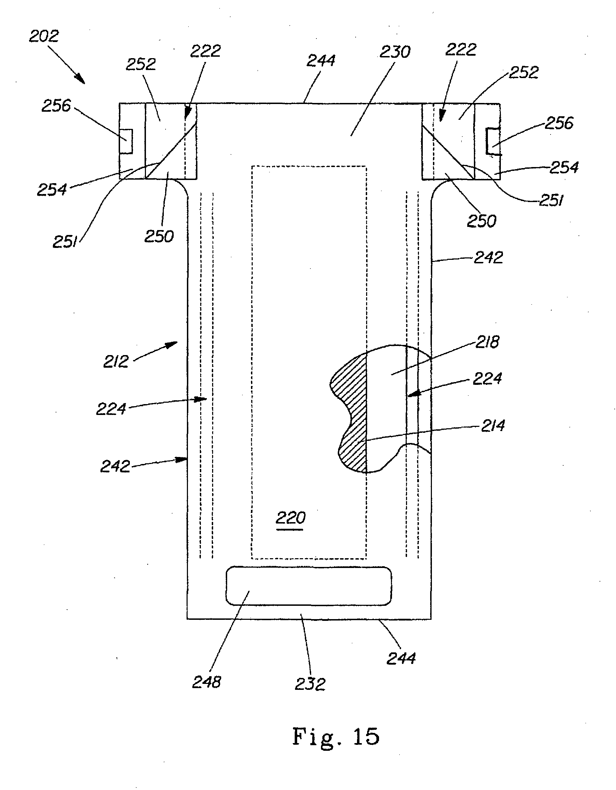

Fig. 15 shows a certain embodiment of a diaper 202, which includes a chassis 212. The chassis 212 has end edges 244 and longitudinal edges 242. The chassis 212 includes topsheet 218, bottom sheet 220, and an absorbent core 214 located between the topsheet 218 and the bottom sheet 220.

The absorbent core 214 is substantially cellulose free and includes an absorbent particulate polymer material. The absorbent core 214 comprises first and second absorbent layers as described hereinabove and made as described hereinbelow. The first absorbent layer includes a first substrate and the second absorbent layer includes a second substrate, with the first and second absorbent layers further including the absorbent particulate polymer material deposited on the first and second substrates and thermoplastic material covering the absorbent particulate polymer material on the respective first and second substrates. The first and second absorbent layers are combined together such that at least a portion of the thermoplastic material of the first absorbent layer contacts at least a portion of the thermoplastic material of the second absorbent layer, and the absorbent particulate polymer material is disposed between the first and second substrates in an absorbent particulate polymer material area. The absorbent particulate polymer material is substantially continuously distributed across the absorbent particulate polymer material area.

The chassis 212 further includes a pair of side panels 222 joined with the chassis 212. The side panels 222 extend laterally outwardly beyond the longitudinal edges 242, each side panel 222 having at least one zone elastically extensible in a direction having a vector component in the lateral direction.

The side panels 222 may have a number of different sizes and shapes. In one embodiment, shown in Fig. 15, the side panels 222 have a rectangular shape. For a typical "large" (8 kg to 14 kg) baby diaper, the side panels may, for example, have a size of about 63 mm in the lateral direction by about 80 mm in the longitudinal direction.

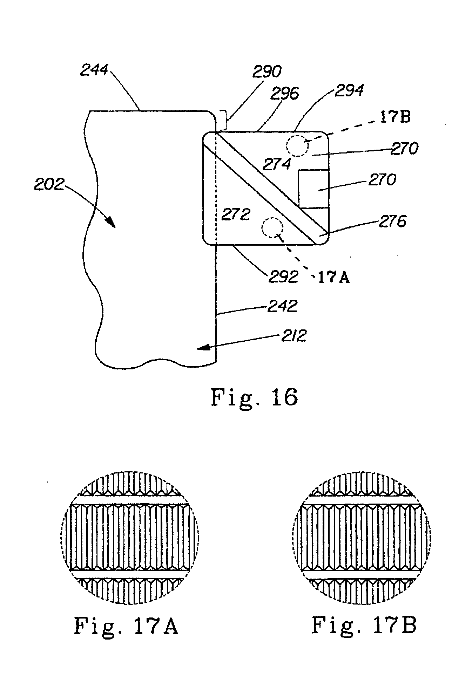

The side panels 222 may be constructed in a number of configurations and from a number of different materials. The side panels may comprise conventional elastic materials or mechanically stretched laminates such as a zero strain stretch laminate. In a certain embodiment, the side panels comprise a SELF web as described in U.S. Pat. No. 5,518,801 issued to Chappell et al. Figs. 17A and 17B show a SELF web forming zones of the side panel 270.

Methods for imparting elasticity to a laminate web are described, for example, in U.S. Patent No. 5,143,679 to Weber et al., U.S. Patent No. 5,156,793 to Buell et al., and in U.S. Patent No. 5,167,897 to Weber et al.

The side panels 222 may comprise a separate element affixed to the chassis 212, or can be constructed as an extension of other elements of the diaper such as the backsheet 220 or the topsheet 218, or both the topsheet 218 and the backsheet 220. In the embodiment shown in Fig. 15, the side panels 222 each comprise a separate web joined to the chassis 212. The side panels 222 may be joined to the chassis 212 in the back waist region and extend laterally outwardly beyond the longitudinal edges 242.

The side panels 222 may have multiple zone with different elastic extensibilities. For example, U.S. Patent No. 6,132,411 to Huber et al., which is incorporated herein, describes certain embodiments of multiple zone side panels. The side panels 222 have a multiple zone construction comprising at least a leg zone 250 and a waist zone 252. The leg zone 250 is positioned adjacent to the longitudinal edge 242 of the chassis 212. The waist zone 252 is positioned laterally outwardly from the leg zone 250. The leg zone 250 is elastically extensible in a direction having a vector component in the lateral direction, such as in the lateral direction. The waist zone 252 is elastically extensible in a direction having a vector component in the lateral direction, such as in the lateral direction. In one embodiment, the leg zone 250 is designed to have a higher or lower extension force than the waist zone 252, providing an extension force differential. The extension force of the leg zone 250 may be, in one embodiment, as low as possible. In a certain embodiment, the extension force of the leg zone 250 may be less than about 0,098 N/cm (10 g/cm), such as less than about 0,099N/cm (5 g/cm), at 50% extension. In one embodiment, the leg zone 250 of each side panel may have an extension force between about 0,019N/cm (2 g/cm) and about 0,098 N/cm(10 g/cm) at 50% extension. In a certain embodiment, the extension force of the waist zone 252 may be between about 0,098 N/cm(10 g/cm) to about 0,294N/cm(30 g/cm), such as between about 0,147N/cm (15 g/cm) and about 0,196N/cm (20 g/cm), at 50% extension.

In the embodiment shown in Fig. 15, the leg zone 250 is separated from the waist zone 252 by a separation zone 251. The separation zone 251 may be non-extensible, may be elongatable only (i.e., inelastic), or may be extensible with an extensiblity that is different from that of the waist zone or the leg zone. The separation zone 251 may be a line of demarcation distinguishing the leg zone 250 from the waist zone 252. In one embodiment, the separation zone 251 is a non-extensible zone that physically separates the leg zone 250 from the waist zone 252. The non-extensible separation zone 251 may be formed with the use of heat/pressure bonds, ultrasonic bonding or mechanical bonding which bonds the various layers of tile side panels together in such a way to render them non-extensible (i.e., not elastically extensible). The separation zone 251 may also be formed by joining additional materials to the side panel to render that portion non-extensible.

The side panels 222 each include a grasping zone 254 disposed laterally outwardly from the waist zone 252. The grasping zone 254 may be a stiff material which is non-extensible and functions to allow for convenient grasping of the side panel 212 by the user to apply the fastening members 256 to the reinforcing strip 248.

In the embodiment shown in Fig. 15, the leg zone 250, the waist zone 252, the separation zone 251, and the grasping zone 254 each comprise the same material, although each zone has its own unique degree of force/extension. The use of a SELF web such as described in the aforementioned U.S. Pat. No. 5,518,801 issued to Chappell et al. allows the force/extension properties of each zone to be specifically designed.

The leg zone 250 may have a number of different sizes and shapes. In the embodiment shown in Fig. 15, the leg zone 250 has a triangular shape. The waist zone 252 also may have a number of different sizes and shapes. In the embodiment shown in Fig. 15, the waist zone 252 has a triangular shape.

The leg zone 250 having the low force and unique triangular shape allows the side panel 222 to expand with the leg movement of the wearer at relatively low forces. This feature provides the benefit of reduced red marking of the wearer's skin, as less force lower tensions are riding along the leg of the wearer. The waist zone 252 maintains a continuous ring of high force in the waist area of the diaper which provides improved initial and sustained fit. This high force zone extends across the end edge of the diaper 202 providing the ring of tension necessary to initially fit and sustain this fit through use.

As shown in Fig. 15, the leg zone 250 may form no portion of the upper end edge of the side panel 222, while the waist zone 252 forms no portion of the lower end edge of the side panel 222.

Fig. 16 shows an alternative embodiment of a side panel 270 (of a pair a like panels). Side panel 270 may be a separate member joined to the chassis 212. The side panel 270 extends laterally outwardly beyond the longitudinal edge 242 of the chassis 212. The side panel 270 includes a leg zone 272, a waist zone 274, and a separation zone 276. The fastening member 278 is joined to the waist zone 274 as the side panel 270 does not include a grasping zone. The leg zone 272 has a triangular shape where a side of the triangle forms a substantial portion of the second (lower) edge 292. The waist zone 274 has a triangular shape where a side of the triangle forms a substantial portion of the first (upper) end edge 294. The side panel 270 is joined to the chassis 212 such that it is spaced away from the end edge 244 of the chassis 212. The first end edge 294 is spaced away from the end edge 244 a distance indicated as 290. Distance 290 is less than about 2 cm, such as less than about 1 cm. The first end edge 294 of the side panel 270 effectively forms a portion of the end edge 296 of the diaper 202.

In an alternative embodiment (not shown), the leg zone forms a portion of both the upper and lower end edges. In another alternative embodiment (not shown), the leg zone forms a portion of both the upper and lower end edges while the waist zone forms a portion of the upper end edge but forms no portion of the lower end edge. In still another alternative embodiment (not shown), the side panels may be designed such that the waist zone forms a portion of both the upper end edge and the lower end edge while the leg zone forms a portion of the lower end edge but forms no portion of the upper end edge.

The side panels 222 may be joined to the chassis 212 in a number of different ways as are known in the art including by adhesives, heat/pressure bonds, ultrasonic bonding, or mechanical bonding. Side panels 222 may be bonded to the chassis 212 via mechanical bonding, as shown in Fig. 15.

In one embodiment, the absorbent article is a diaper that may further include an extensible waist feature. The extensible waist features may extend longitudinally outwardly from the chassis, such as a respective waist edge, and generally form at least a portion of the end edge of the diaper. The waist feature may be constructed as a separate element joined to the chassis or as an extension of other elements of the diaper (i.e., unitary). The waist feature may be constructed as an extension of other elements of the chassis, such as the backsheet, the topsheet, or both.

As shown in Fig. 15, the diaper 202 may be provided with an extensible back waist feature 230. The extensible back waist feature 230 may provide an extensible member that provides a more comfortable and contouring fit by initially conformably fitting the diaper to the wearer and sustaining this fit throughout the time of wear well past when the diaper has been loaded with exudates since the extensible back waist feature allows the diaper to expand and, in one embodiment, to contract. Further, the extensible back waist feature 230 develops and maintains wearing forces (tensions) that enhance the tensions developed and maintained by the closure system to maintain the diaper on the wearer and that enhance the fit of the diaper about the waist of the wearer. The extensible back waist feature 230 may further provide more effective application of the diaper since even if the diaperer pulls one side panel of the extensible back waist feature farther than the other during application (asymmetrically), the diaper will "self-adjust" during wear.

As shown in Fig. 15, the diaper 202 may also be provided with an extensible front waist feature 232. The extensible front waist feature 232 is designed to fit around the abdomen in the front waist of the wearer to improve the fit and containment of the diaper at the front waist. The extensible front waist feature 232 extends longitudinally outwardly from the chassis 212, typically the waist edge of the absorbent core 214, and generally forms at least a portion of the end edge 244 of the diaper 202 in the front waist region.

The extensible front waist feature 232 may comprise any of the known configurations of an elastic feature or any of the elastically extensible features as described herein. The extensible back waist feature may be constructed in a number of configurations and from a number of different materials. For example, the extensible back waist feature may be elasticized by operatively joining an elastic member thereto such as the elasticized waistbands known in the art and as are disclosed in U.S. Pat. No. 4,515,595 issued to Kievit, et al., U.S. Pat. No. 5,151,092 issued to Buell et al., and U.S. Pat. No. 5,628,741 issued to Buell et al.. Thus, the extensible back waist feature may be a stretch laminate such as a zero strain stretch laminate, such as described in U.S. Pat. No. 5,151,092 Buell, et al. In a certain embodiment, the extensible back waist feature comprises a structural elastic-like film SELF web as described in U.S. Pat. No. 5,518,801 issued to Chappell et al.. A method of modifying a nonwoven fibrous web for use as a component, such as a side panel, of a disposable absorbent article is described in U.S. Patent No. 6,383,431 to Dobrin et al..

The web material may include a strainable network having at least two distinct and dissimilar regions comprised of the same material composition. The first region is oriented substantially parallel to an axis of the elongation such that it will undergo a molecular-level deformation in response to an applied axial elongation in a direction substantially parallel to elongation axis before a substantial portion of the second region undergoes any substantial molecular-level deformation. As used herein, the term "substantially parallel" refers to an orientation between two axes whereby the subtended angle formed by the two axes or an extension of the two axes is less than 45°. In the case of a curvilinear element, it may be more convenient to use a linear axis which represents an average of the curvilinear element. The second regions initially undergo a substantially geometric deformation in response to an applied elongation in a direction substantially parallel to the axis.

The chassis 212 may further extensible leg cuffs 224 for providing improved containment of liquids and other body exudates. Each extensible leg cuff 224 may comprise several different embodiments for reducing the leakage of body exudates in the leg regions. (The leg cuff can be and is sometimes also referred to as leg bands, leg flaps, barrier cuffs, or elastic cuffs.) U.S. Pat. No. 3,860,003 issued to Buell on Jan. 14, 1975, describes a disposable diaper which provides a contractible leg opening having a leg flap and one or more elastic members to provide an elasticized leg cuff (gasketing cuff). U.S. Pat. No. 4,909,803 issued to Aziz et al. on Mar. 20, 1990, describes a disposable diaper having "stand-up" elasticized flaps (barrier cuffs) to improve the containment of the leg regions. U.S. Pat. No. 4,695,278 issued to Lawson on Sep. 22, 1987, describes a disposable diaper having dual cuffs including a gasketing cuff and a barrier cuff. U.S. Pat. No. 4,704,115 issued to Buell on Nov. 3, 1987, discloses a disposable diaper or incontinent garment having side-edge-leakage-guard gutters configured to contain free liquids within the garment. U.S. Pat. No. 5,032,120 issued to Freeland et al. on Jul. 16, 1991, discloses an absorbent article having leg cuffs having a relatively low ultimate contact force at relatively high elongations accomplished, for example, by low contact force differential material. U.S. Pat. No. 5,087,255 issued to Sims on Feb. 11, 1992, discloses an absorbent article having inflected barrier cuffs with the distal edge positioned outboard of the proximal edge in one waist region and inboard in the other to provide better fit about the hips/buttocks.

The diaper 202 may also be provided with a closure system for fitting the diaper on the wearer. While the closure system may take on a number of configurations such as adhesive tape tabs, mechanical closure tape tabs, fixed position fasteners, side seams as for training pants, or any other closure means as are known in the art. As shown in Fig. 16, the closure system may include an adhesive tape tab fastening system including a pair of tape tab fastening members 256 and a landing member, such as a reinforcing strip 248 as in Fig. 15 or, in the alternative, a portion of the backsheet, positioned in the front waist region of the diaper 202. Examples of suitable adhesive tape tab fastening systems are disclosed in U.S. Pat. No. 3,848,594 issued to Buell on Nov. 19, 1974; and U.S. Pat. No. 4,662,875 issued to Hirotsu and Robertson on May 5, 1987. Examples of other closure systems, including mechanical closure systems, useful in the present invention, are disclosed in U.S. Pat. No. 4,869,724 issued to Scripps on Sep. 26, 1989; U.S. Pat. No. 4,848,815 issued to Scripps on Jul. 11, 1989; and the two-point fastening system described in U.S. Pat. No. 5,242,436 issued to Weil, Buell, Clear, and Falcone on Sep. 7, 1993.

The diaper 202 may be applied to a wearer by positioning the back waist region under the wearer's back and drawing the remainder of the diaper between the wearer's legs so that the front waist region is positioned across the front of the wearer. The tab portions of the tape tabs 256 are then released from the release portion. The diaperer then wraps the side panel 222 around the wearer; while still grasping the tab portion. The side panel will typically be extended and tensioned during this operation so as to conform to the size and shape of the wearer. The tape tab 256 is secured to the reinforcing strip 248, i.e., the landing member, on the chassis 212 to effect a side closure. The process is then repeated with the other tape tab. Thus, the diaper is closed on the wearer and the extensible back waist feature and the other elements, if provided, provide the fit and containment benefits as described herein. Alternatively, the diaper may be fastened prior to being fitted on the wearer such that it may be used as a pant. In either configuration, the pant or diaper may be removed by disengaging the fasteners or by removing it as one would a pant.

In a certain embodiment, the disposable absorbent article is a disposable training pant having absorbent core which is substantially cellulose free and elastically extensible side panels for improved fit and comfort. Examples of constructing the side panels and chassis are described in U.S. Patent No. 5,246,433 to Hasse et al. and in U.S. Patent No. 5,591,155 to Nishikawa et al..

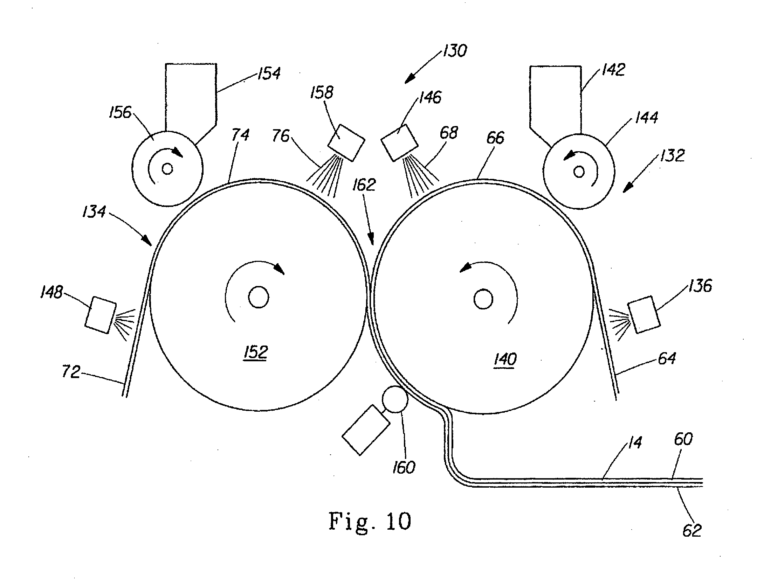

A printing system 130 for making an absorbent core 14 in accordance with an embodiment of this invention is illustrated in Fig. 10 and may generally comprise a first printing unit 132 for forming the first absorbent layer 60 of the absorbent core 14 and a second printing unit 134 for forming the second absorbent layer 62 of the absorbent core 14.

The first printing unit 132 may comprise a first auxiliary adhesive applicator 136 for applying an auxiliary adhesive to the substrate 64, which may be a nonwoven web, a first rotatable support roll 140 for receiving the substrate 64, a hopper 142 for holding absorbent particulate polymer material 66, a printing roll 144 for transferring the absorbent particulate polymer material 66 to the substrate 64, and a thermoplastic adhesive material applicator 146 for applying the thermoplastic adhesive material 68 to the substrate 64 and the absorbent particulate polymer 66 material thereon.

The second printing unit 134 may comprise a second auxiliary adhesive applicator 148 for applying an auxiliary adhesive to the second substrate 72, a second rotatable support roll 152 for receiving the second substrate 72, a second hopper 154 for holding the absorbent particulate polymer material 74, a second printing roll 156 for transferring the absorbent particulate polymer material 74 from the hopper 154 to the second substrate 72, and a second thermoplastic adhesive material applicator 158 for applying the thermoplastic adhesive material 76 to the second substrate 72 and the absorbent particulate polymer material 74 thereon.

The printing system 130 also includes a guide roller 160 for guiding the formed absorbent core from a nip 162 between the first and second rotatable support rolls 140 and 152.

The first and second auxiliary applicators 136 and 148 and the first and second thermoplastic adhesive material applicators 146 and 158 may be a nozzle system which can provide a relatively thin but wide curtain of thermoplastic adhesive material.

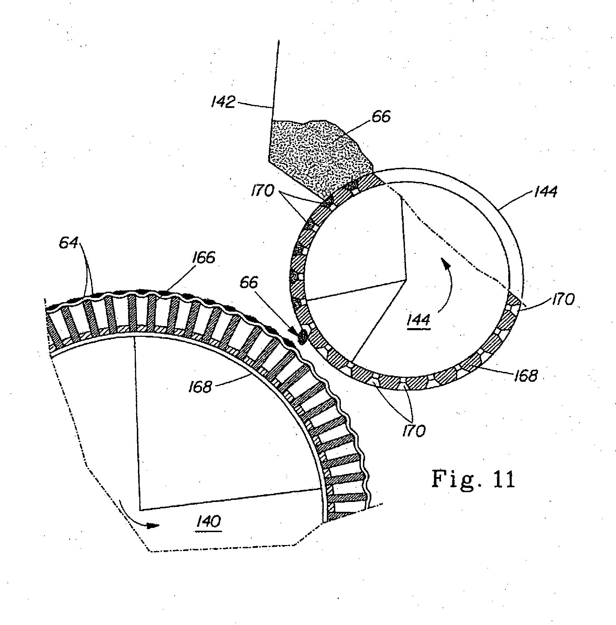

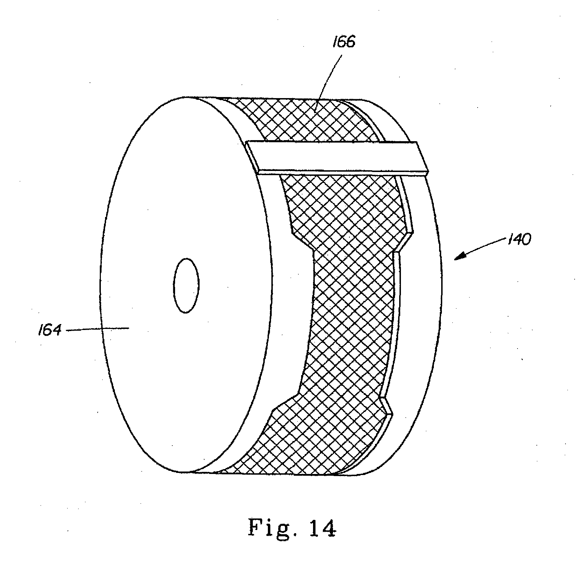

Turning to Fig. 11, portions of the first hopper 142, first support roll 140, and first printing roll 144 are illustrated. As also shown in Fig. 14, the first rotatable support roll 140, which has the same structure as the second rotatable support roll 152, comprises a rotatable drum 164 and a peripheral vented support grid 166 for receiving the first substrate 64.

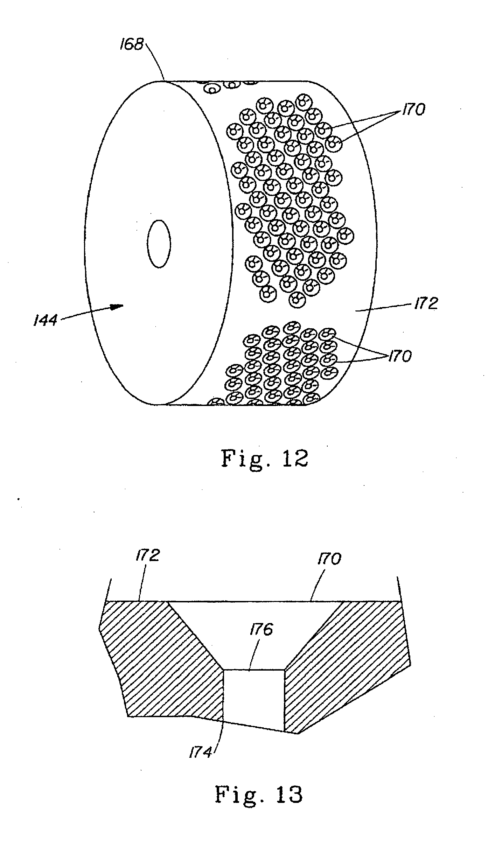

As also illustrated in Fig. 12, the first printing roll 144, which has the same structure as the second printing roll 156, comprises a rotatable drum 168 and a plurality of absorbent particulate polymer material reservoirs 170 in a peripheral surface 172 of the drum 168. The reservoirs 170 best illustrated in Fig. 13, may have a variety of shapes, including cylindrical, conical, or any other shape. The reservoirs 170 may lead to an air passage 174 in the drum 168 and comprise a vented cover 176 for holding adhesive particulate polymer material 66 in the reservoir and preventing the adhesive particulate polymer material 66 from falling or being pulled into the air passage 174.

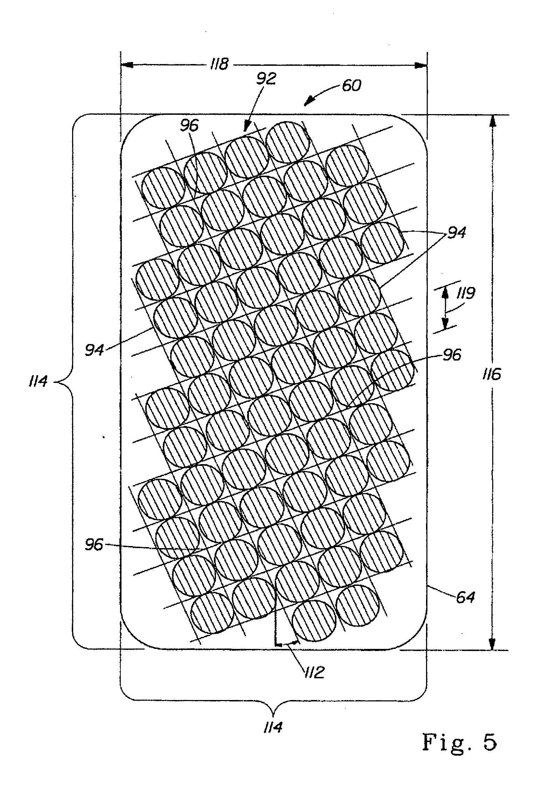

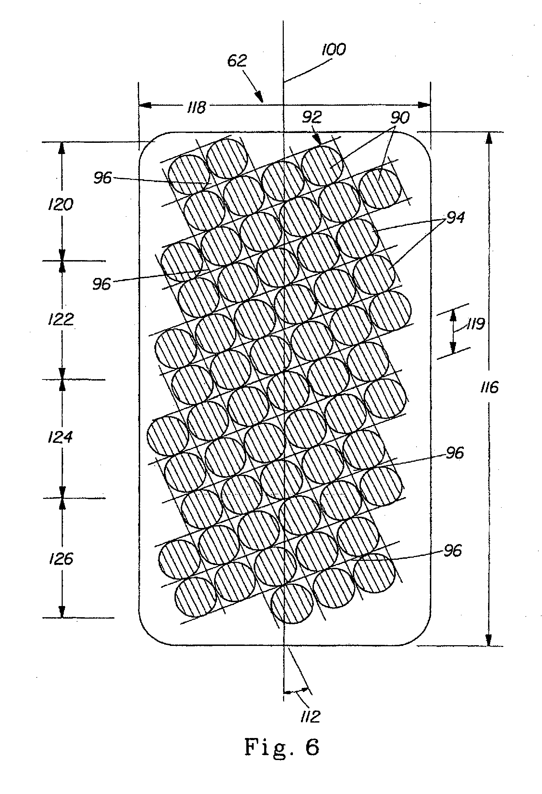

In operation, the printing system 130 receives the first and second substrate 64 and 72 into the first and second printing units 132 and 134, respectively, the first substrate 64 is drawn by the rotating first support roll 140 past the first auxiliary adhesive applicator 136 which applies the first auxiliary adhesive to the first substrate 64 in a pattern such as described hereinabove. A vacuum (not shown) within the first support roll 140 draws the first substrate 64 against the vertical support grid 166 and holds the first substrate 64 against the first support roll 140. This presents an uneven surface on the first substrate 64. Due to gravity, or by using the vacuum means, the substrate 64 will follow the contours of the uneven surface and thereby the substrate 64 will assume a mountain and valley shape. The absorbent particulate polymer material 66 may accumulate in the valleys presented by the substrate 64. The first support roll 140 then carries the first substrate 64 past the rotating first printing roll 144 which transfers the absorbent particulate polymer material 66 from the first hopper 142 to the first substrate 64 in the grid pattern 92 which is best illustrated in Figs. 5 and 6. A vacuum (not shown) in the first printing roll 144 may hold the absorbent particulate polymer material 66 in the reservoirs 170 until time to deliver the absorbent particulate polymer material 66 to the first substrate 64. The vacuum may then be released or air flow through the air passages 174 may be reversed to eject the absorbent particulate polymer material 66 from the reservoirs and onto the first substrate 64. The absorbent particulate polymer material 66 may accumulate in the valleys presented by the substrate 64. The support roll 140 then carries the printed first substrate 64 past the thermoplastic adhesive material applicator 136 which applies the thermoplastic adhesive material 68 to cover the absorbent particulate polymer material 66 on the first substrate 64.

Hence, the uneven surface of the vented support grid 166 of the support rolls 140 and 152 determines the distribution of absorbent particulate polymeric material 66 and 74 throughout the absorbent core 14 and likewise determines the pattern of junction areas 96.

Meanwhile, the second rotatable support roll draws the second substrate 72 past the second auxiliary adhesive applicator 148 which applies an auxiliary adhesive to the second substrate 72 in a pattern such as is described hereinabove. The second rotatable support roll 152 then carries the second substrate 72 past the second printing roll 156 which transfers the absorbent particulate polymer material 74 from the second hopper 154 to the second substrate 72 and deposits the absorbent particulate polymer material 74 in the grid pattern 92 on the second substrate 72 in the same manner as described with regard to the first printing unit 132 above. The second thermoplastic adhesive material applicator 158 then applies the thermoplastic adhesive material 76 to cover the absorbent particulate polymer material 74 on the second substrate 72. The printed first and second substrates 64 and 72 then pass through the nip 162 between the first and second support rolls 140 and 152 for compressing the first absorbent layer 60 and second absorbent layer 62 together to form the absorbent core 14.

In an optional further process step a cover layer 70 may be placed upon the substrates 64 and 72, the absorbent particulate polymer material 66 and 74, and the thermoplastic adhesive material 68 and 76. In another embodiment, the cover layer 70 and the respective substrate 64 and 72 may be provided from a unitary sheet of material. The placing of the cover layer 70 onto the respective substrate 64 and 72 may then involve the folding of the unitary piece of material.

The test method and apparatuses described below may be useful in testing embodiments of this invention:

### Wet Immobilization Test

### Equipment

- Graduated Cylinder
- Stop watch (± 0.1 sec)
- Scissors
- Light Box
- Pen
- Test solution: 0.90% saline solution at 37°C
- Metal ruler traceable to NIST, DIN, JIS or other comparable National Standard
- PVC/metal dishes with a flat surface inside and a minimum length of the core bag length (n) to be measured and a maximum length n + 30mm, width of 105 ± 5 mm, height of 30-80 mm or equivalent
- Electronic Force Gauge (Range 0 to 50 Kg)
- Wet Immobilization Impact Tester Equipment (WAIIT), Design package number: BM-00112.59500-R01 available from T.M.G. Technisches Buero Manfred Gruna

### Facilities:

Standard laboratory conditions, temperature: 23°C ±2°C, relative humidity: < 55%

### Sample Preparation

1. Open the product, topsheet side up.
2. Unfold the diaper and cut the cuff elastics approximately every 2.5 cm to avoid chassis tension.
3. For pull-up products open the side seams and remove the waistbands.
4. Lay the core bag flat and rectangular topsheet side up onto the light box surface without any folds.
5. Switch on the light box to clearly identify the absorbent core outer edges.
6. With a ruler, draw a line at the front and back absorbent core outer edges.
7. Measure the distance (A), between the two markers and divide the value by 2, this will be calculated distance (B).
8. Measure the calculated distance (B) from front marker towards the middle of the core bag and mark it. At this marker draw a line in the cross direction.

### Test Procedure

### WAIIT Calibration:

1. Make sure that the sliding board is in the lower position. Open the front door of the WAIIT tester and connect the force gauge hook to the upper sample clamp of the WAIIT. Make sure that the clamp is closed before connecting the spring-balance.
2. Use both hands on the spring-balance to lift continuously and as slowly as possible up the sliding board towards the upper position. Record the average value (m₁) during the execution to the nearest 0.02 kg.
3. Guide down the sliding board as slowly as possible to the lower position and record the average value (m₂) read off during execution to the nearest 0.02 kg.
4. Calculate and report the delta of m₁ - m₂ to the nearest 0.01 kg. If the delta is 0.6 kg ± 0.3 kg continue measurement. Otherwise, an adjustment of the sliding board is necessary. Make sure that the sliding board is in lower position and check the sliding path for any contamination or damage. Check if the position of the sliding board to the sliding path is correctly adjusted by shaking the board. For easy gliding some clearance is needed. If not present, readjust the system.

### WAIIT test settings:

- Drop height is 50 cm.
- Diaper load (l_{D}) is 73% of the core capacity (cc); l_{D} = 0.73 x cc.
- Core capacity (cc) is calculated as: cc = m_{SAP} x SAP_{GV}. where m_{SAP} is the mass of superabsorbent polymer (SAP) present in the diaper and SAP_{GV} is the free swelling capacity of the superabsorbent polymer. Free swelling capacity of the superabsorbent polymer is determined with the method described in WO 2006/062258. The mass of the superabsorbent polymer present in the diaper is the average mass present in ten products.

### Test execution:

1. Reset the balance to zero (tare), put the dry core bag on the balance, weigh and report it to the nearest 0.1 g.
2. Measure the appropriate volume Saline (0.9% NaCl in deionized water) with the graduated cylinder.
3. Lay the core bag, topsheet side up, flat into the PVC dish. Pour the saline evenly over the core bag.
4. Take the PVC dish and hold it slanting in different directions, to allow any free liquid to be absorbed. Products with poly-backsheet need to be turned after a minimum waiting time of 2 minutes so that liquid under the backsheet can be absorbed. Wait for 10 minutes (+/- 1 minute) to allow all saline to be absorbed. Some drops may retain in the PVC dish. Use only the defined PVC/metal dish to guarantee homogenous liquid distribution and less retained liquid.
5. Reset the balance to zero (tare), put the wet core bag on the balance. Weigh and report it to the nearest 0.1 g. Fold the core bag just once to make it fit on the balance. Check to see if the wet core bag weight is out of limit (defined as "dry core bag weight + diaper load ± 4 ml"). For example, 12 g dry core bag weight + 150 ml load = 162 g wet core bag weight. If the actual wet weight on the scale is between 158g and 166g, the pad can be used for shaking. Otherwise scrap the pad and use the next one.
6. Take the loaded core bag and cut the pad along the marked line in the cross direction.
7. Put the back of the wet core bag onto the balance (m₁). Weigh and report it to the nearest 0.1 g.
8. Take the wet core and clamp the end seal side in the top clamp of the sample holder of the WAIIT (open end of the core oriented down). Next, clamp both sides of the core with the side clamps of the sample holder making sure that the product is fixed to the sample holder along the whole product length. Make sure not to clamp the absorbent core, only the nonwoven; for some products this means securing the product with only the barrier leg cuff.
9. Lift up the sliding board to the upper position by using both hands until the board is engaged.
10. Close the safety front door and release the slide blade.
11. Reset the balance to zero (tare), take the tested core bag out of the WAIIT and put it on the balance (m₂). Report the weight to the nearest 0.1 g.
12. Repeat steps 7 to 11 with front of the wet core bag.

Reporting:
1. Record the dry core bag weight to the nearest 0.1 g.
2. Record the wet weight before (m_{1 front/back}) and after (m_{2 front/back}) testing, both to the nearest 0.1 g.
3. Calculate and report the average weight loss (Δm) to the nearest 0.1 g: Δm = (m_{1front} + m_{1back}) - (m_{2front} + m_{2back})
4. Calculate and report the weight loss in percent to the nearest 1%, (Δmᵣₑₗ): (Δmᵣₑₗ) = (((m_{1front} + m_{1back}) - (m_{2front} + m_{2back})) x 100) /(m_{1front} + m_{1back})
5. Calculate and report Wet Immobilization (WI) as: WI = 100% - Δmᵣₑₗ

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. A disposable absorbent article comprising:
a chassis including a topsheet and a backsheet, the chassis having end edges and longitudinal edges;
an absorbent core which comprises an absorbent particulate polymer material, the absorbent core being located between the topsheet and the backsheet; and
a pair of side panels joined with the chassis, the side panels extending laterally outwardly beyond the longitudinal edges, each side panel having at least one zone elastically extensible in a direction having a vector component in the lateral direction,
wherein each side panel has a leg zone which is elastically extensible in a direction having a vector component in the lateral direction, a waist zone which is elastically extensible in a direction having a vector component in the lateral direction, and a separation zone separating the leg zone and the waist zone, and **characterized in that** the absorbent core is substantially cellulose free and comprises first and second absorbent layers, the first absorbent layer including a first substrate and the second absorbent layer including a second substrate, the first and second absorbent layers further including the absorbent particulate polymer material deposited on said first and second substrates and thermoplastic adhesive material covering the absorbent particulate polymer material on the respective first and second substrates, said first and second absorbent layers combined together such that at least a portion of said thermoplastic adhesive material of said first absorbent layer contacts at least a portion of the thermoplastic adhesive material of said second absorbent layer, the absorbent particulate polymer material is disposed between the first and second substrates in an absorbent particulate polymer material area, wherein the absorbent particulate polymer material is deposited on the first and second substrates in respective patterns of land areas and junction areas between the land areas such that the absorbent particulate polymer material is discontinuously distributed on the first and second substrates, and the first and second absorbent layers are combined together such the respective patterns of absorbent particulate polymer material are offset from one another, and the absorbent particulate polymer material is substantially continuously distributed across the absorbent particulate polymer material area.

2. The disposable absorbent article of claim 1, wherein the separation zone is non-elastically extensible.

3. The disposable absorbent article according to any one of the preceding claims, wherein each side panel further comprises a grasping zone disposed laterally outwardly from the waist zone.

4. The disposable absorbent article according to any one of the preceding claims, wherein the leg zone and the waist zone have an extension force differential.

5. The disposable absorbent article according to any one of the preceding claims, wherein the leg zone of each side panel has an extension force between 0,019N/cm (2 g/cm) and 0,098N/cm (10 g/cm) at 50% extension, wherein the waist zone of each side panel has an extension force between 10 g/cm and 30 g/cm at 50% extension.

6. The disposable absorbent article according to any of the preceding claims, wherein the absorbent core has a length extending from a rear end to a front end, and a width extending from a first edge to a second edge and perpendicularly to the length, and the respective patterns are offset from one another in both a direction parallel to the length and a direction parallel to the width.

7. The disposable absorbent article according to any of the preceding claims, wherein the absorbent core has a front end and a rear end and a longitudinal axis extending from the rear end to the front end and a plurality of absorbent zones, each of the plurality of absorbent zones having absorbent particulate polymer material present in differing amounts and a gradual transition in amount of absorbent particulate polymer material form one of the plurality of absorbent zones to another.

8. The disposable absorbent article according to any one of the preceding claims, wherein the absorbent article is a diaper and the pair of side panels comprise a re-closable fastening system for securing the diaper to a wearer.

9. The disposable absorbent article according to any one of the preceding claims, wherein the absorbent article is a pant-type diaper and the pair of side panels is joined to each other to form a pant.

## Patentansprüche

1. Einwegabsorptionsartikel, umfassend:
eine Grundeinheit, die eine Oberschicht und eine Unterschicht einschließt, wobei die Grundeinheit Endränder und Längsränder aufweist;
einen Absorptionskern, der ein Polymerteilchen-Absorptionsmaterial umfasst, wobei der Absorptionskern zwischen der Oberschicht und der Unterschicht angeordnet ist; und
ein Paar Seitenfelder, die mit der Grundeinheit verbunden sind, wobei sich die Seitenfelder über die Längsränder hinaus seitlich nach außen erstrecken, wobei jedes Seitenfeld mindestens eine Zone aufweist, die in einer Richtung mit einer Vektorkomponente in der Querrichtung elastisch dehnbar ist,
wobei jedes Seitenfeld eine Beinzone, die in einer Richtung mit einer Vektorkomponente in der Querrichtung elastisch dehnbar ist, eine Taillenzone, die in einer Richtung mit einer Vektorkomponente in der Querrichtung elastisch dehnbar ist, und eine Trennzone, die die Beinzone und die Taillenzone trennt, aufweist, und **dadurch gekennzeichnet, dass** der Absorptionskern im Wesentlichen cellulosefrei ist und eine erste und eine zweite Absorptionsschicht umfasst, wobei die erste Absorptionsschicht ein erstes Substrat einschließt und die zweite Absorptionsschicht ein zweites Substrat einschließt, wobei die erste und die zweite Absorptionsschicht ferner das Polymerteilchen-Absorptionsmaterial einschließen, das auf dem ersten und dem zweiten Substrat angelagert ist, und thermoplastisches Klebstoffmaterial, welches das Polymerteilchen-Absorptionsmaterial auf dem ersten bzw. zweiten Substrat abdeckt, wobei die erste und die zweite Absorptionsschicht so miteinander kombiniert sind, dass wenigstens ein Teil des thermoplastischen Klebstoffmaterials der ersten Absorptionsschicht wenigstens einen Teil des thermoplastischen Klebstoffmaterials der zweiten Absorptionsschicht berührt, wobei das Polymerteilchen-Absorptionsmaterial zwischen dem ersten und dem zweiten Substrat in einem Polymerteilchen-Absorptionsmaterialbereich angeordnet ist, wobei das Polymerteilchen-Absorptionsmaterial auf dem ersten und dem zweiten Substrat in jeweiligen Mustern von Stegflächen und Verbindungsflächen zwischen den Stegflächen angelagert ist, so dass das Polymerteilchen-Absorptionsmaterial diskontinuierlich auf dem ersten und zweiten Substrat verteilt ist, und wobei die erste und die zweite Absorptionsschicht so miteinander kombiniert sind, dass die jeweiligen Muster von Polymerteilchen-Absorptionsmaterial zueinander versetzt sind und das Polymerteilchen-Absorptionsmaterial im Wesentlichen kontinuierlich über den Polymerteilchen-Absorptionsmaterialbereich verteilt ist.

2. Einwegabsorptionsartikel nach Anspruch 1, wobei die Trennzone nichtelastisch dehnbar ist.

3. Einwegabsorptionsartikel nach einem der vorstehenden Ansprüche, wobei jedes Seitenfeld ferner eine Greifzone umfasst, die von der Taillenzone seitlich nach außen angeordnet ist.

4. Einwegabsorptionsartikel nach einem der vorstehenden Ansprüche, wobei die Beinzone und die Taillenzone ein Dehnkraftdifferenzial aufweisen.

5. Einwegabsorptionsartikel nach einem der vorstehenden Ansprüche, wobei die Beinzone jedes Seitenfelds eine Dehnkraft zwischen 0,019 N/cm (2 g/cm) und 0,098 N/cm (10 g/cm) bei 50 % Dehnung aufweist, wobei die Taillenzone jedes Seitenfelds eine Dehnkraft zwischen 0,098 N/cm (10 g/cm) und 0,294 N/cm (30 g/cm) bei 50 % Dehnung aufweist.

6. Einwegabsorptionsartikel nach einem der vorstehenden Ansprüche, wobei der Absorptionskern eine Länge, die von einem hinteren Ende zu einem vorderen Ende verläuft, und eine Breite, die von einem ersten Rand zu einem zweiten Rand und senkrecht zur Länge verläuft, aufweist, und wobei die jeweiligen Muster sowohl in einer Richtung parallel zur Länge als auch in einer Richtung parallel zur Breite zueinander versetzt sind.

7. Einwegabsorptionsartikel nach einem der vorstehenden Ansprüche, wobei der Absorptionskern ein vorderes Ende und ein hinteres Ende und eine Längsachse, die vom hinteren Ende zum vorderen Ende verläuft, und eine Vielzahl von Absorptionszonen aufweist, wobei in jeder von der Vielzahl von Absorptionszonen Polymerteilchen-Absorptionsmaterial in unterschiedlichen Mengen vorhanden ist und ein allmählicher Übergang der Menge an Polymerteilchen-Absorptionsmaterial von einer von der Vielzahl von Absorptionszonen zu einer anderen stattfindet.

8. Einwegabsorptionsartikel nach einem der vorstehenden Ansprüche, wobei der Absorptionsartikel eine Windel ist und das Paar Seitenfelder ein wiederverschließbares Befestigungssystem zum Befestigen der Windel an einem Träger umfasst.

9. Einwegabsorptionsartikel nach einem der vorstehenden Ansprüche, wobei der Absorptionsartikel eine hosenartige Windel ist und das Paar Seitenfelder miteinander verbunden ist, um eine Hose zu bilden.

## Revendications

1. Article absorbant jetable comprenant :
un châssis incluant une feuille de dessus et une feuille de fond, le châssis possédant des bords extrêmes et des bords longitudinaux ;
une âme absorbante qui comprend un matériau polymère particulaire absorbant, l'âme absorbante étant située entre la feuille de dessus et la feuille de fond ; et
une paire de pans latéraux joints au châssis, les pans latéraux s'étendant latéralement vers l'extérieur au-delà des bords longitudinaux, chaque pan latéral possédant au moins une zone élastiquement extensible dans une direction possédant une composante vectorielle dans la direction latérale,
dans lequel chaque pan latéral a une zone de jambe qui est élastiquement extensible dans une direction possédant une composante vectorielle dans la direction latérale, une zone de ceinture qui est élastiquement extensible dans une direction possédant une composante vectorielle dans la direction latérale, et une zone de séparation séparant la zone de jambe et la zone de ceinture, et **caractérisé en ce que** l'âme absorbante est essentiellement exempte de cellulose et comprend des première et deuxième couches absorbantes, la première couche absorbante incluant un premier substrat et la deuxième couche absorbante incluant un deuxième substrat, les première et deuxième couches absorbantes incluant en outre le matériau polymère particulaire absorbant déposé sur lesdits premier et deuxième substrats et un matériau adhésif thermoplastique couvrant le matériau polymère particulaire absorbant sur les premier et deuxième substrats respectifs, lesdites première et deuxième couches absorbantes combinées l'une à l'autre de telle sorte qu'au moins une partie dudit matériau adhésif thermoplastique de ladite première couche absorbante vient en contact avec au moins une partie du matériau adhésif thermoplastique de ladite deuxième couche absorbante, le matériau polymère particulaire absorbant est disposé entre les premier et deuxième substrats dans une zone de matériau polymère particulaire absorbant, dans lequel le matériau polymère particulaire absorbant est déposé sur les premier et deuxième substrats dans des motifs respectifs de zones planes et de zones de jonction entre les zones planes de telle sorte que le matériau polymère particulaire absorbant est réparti de façon discontinue sur les premier et deuxième substrats, et les première et deuxième couches absorbantes sont combinées l'une à l'autre de telle sorte que les motifs respectifs de matériau polymère particulaire absorbant sont décalés l'un par rapport à l'autre, et le matériau polymère particulaire absorbant est réparti de façon essentiellement continue à travers la zone de matériau polymère particulaire absorbant.

2. Article absorbant jetable selon la revendication 1, dans lequel la zone de séparation est non élastiquement extensible.

3. Article absorbant jetable selon l'une quelconque des revendications précédentes, dans lequel chaque pan latéral comprend en outre une zone de préhension disposée latéralement vers l'extérieur de la zone de ceinture.

4. Article absorbant jetable selon l'une quelconque des revendications précédentes, dans lequel la zone de jambe et la zone de ceinture présentent une différence de force d'extension.

5. Article absorbant jetable selon l'une quelconque des revendications précédentes, dans lequel la zone de jambe de chaque pan latéral a une force d'extension comprise entre 0,019 N/cm (2 g/cm) et 0,098 N/cm (10 g/cm) à une extension de 50 %, dans lequel la zone de ceinture de chaque pan latéral a une force d'extension comprise entre 0,098 N/cm (10 g/cm) et 0,294 N/cm (30 g/cm) à une extension de 50 %.

6. Article absorbant jetable selon l'une quelconque des revendications précédentes, dans lequel l'âme absorbante a une longueur s'étendant d'une extrémité arrière à une extrémité avant, et une largeur s'étendant d'un premier bord à un deuxième bord et perpendiculairement à la longueur, et les motifs respectifs sont décalés l'un de l'autre à la fois dans une direction parallèle à la longueur et une direction parallèle à la largeur.

7. Article absorbant jetable selon l'une quelconque des revendications précédentes, dans lequel l'âme absorbante a une extrémité avant et une extrémité arrière et un axe longitudinal s'étendant de l'extrémité arrière à l'extrémité avant et une pluralité de zones absorbantes, chacune parmi la pluralité de zones absorbantes ayant un matériau polymère particulaire absorbant présent en quantités différentes et une transition progressive de quantité de matériau polymère particulaire absorbant d'une zone de la pluralité de zones absorbantes à une autre.

8. Article absorbant jetable selon l'une quelconque des revendications précédentes, où l'article absorbant est une couche et la paire de pans latéraux comprend un système de fixation refermable pour fixer la couche sur un porteur.

9. Article absorbant jetable selon l'une quelconque des revendications précédentes, où l'article absorbant est une couche de type culotte et la paire de pans latéraux est jointe mutuellement de façon à former une culotte.
